# EUROPÄISCHE PATENTSCHRIFT

(11) **EP 3 012 759 B1**
(45) Veröffentlichungstag und Bekanntmachung des Hinweises auf die Patenterteilung: **02.10.2019**
(21) Anmeldenummer: 15191121.1
(22) Anmeldetag: 22.10.2015
(51) Int. Cl.: G16H 40/60

(54) **VERFAHREN ZUR PLANUNG, VORBEREITUNG, BEGLEITUNG, ÜBERWACHUNG UND/ODER ABSCHLIESSENDEN KONTROLLE EINES OPERATIVEN EINGRIFFS IN DEN MENSCHLICHEN ODER TIERISCHEN KÖRPER, VORRICHTUNG ZUM AUSFÜHREN EINES SOLCHEN EINGRIFFS UND VERWENDUNG DER VORRICHTUNG**
METHOD FOR PLANNING, PREPARING, ACCOMPANIMENT, MONITORING AND/OR FINAL CONTROL OF A SURGICAL PROCEDURE IN THE HUMAN OR ANIMAL BODY, SYSTEM FOR CARRYING OUT SUCH A PROCEDURE AND USE OF THE DEVICE
PROCÉDÉ DE PLANIFICATION, DE PRÉPARATION, DE SUIVI, DE SURVEILLANCE ET/OU DE CONTRÔLE FINAL D'UNE INTERVENTION OPÉRATOIRE DANS LES CORPS HUMAINS OU D'ANIMAUX, PROCÉDÉ D'EXÉCUTION D'UNE TELLE INTERVENTION ET UTILISATION DU DISPOSITIF

(30) Priorität: 24.10.2014 DE 102014221738
(43) Veröffentlichungstag der Anmeldung: 27.04.2016
(73) Patentinhaber: mediCAD Hectec GmbH, 84032 Altdorf (DE)
(72) Erfinder: SEEL, Jörn, 61440 Oberursel (DE)
(74) Vertreter: LS-MP von Puttkamer Berngruber Loth Spuhler

(56) Entgegenhaltungen:
- WO-A1-2012/135653
- WO-A1-2013/025814
- US-A1- 2014 282 008
- US-A1- 2014 303 493

## Beschreibung

Die Erfindung betrifft ein Verfahren zur Planung, Vorbereitung, Überwachung und/oder abschließenden Kontrolle und Begleitung eines operativen Eingriffs in den menschlichen oder tierischen Körper, insbesondere zum Einsatz wenigstens eines Implantats, bei dem mit Hilfe einer Bildassistenzeinrichtung aus vorhandenen Daten mehrdimensionale Darstellungen zumindest eines Abschnitts des Körpers gezeigt werden, von denen sich wenigstens zwei Darstellungen in Bezug auf die Anzahl der Dimensionen der Darstellung unterscheiden. Die Erfindung betrifft vor allem eine Möglichkeit, in das Verfahren Vorrichtungen zum Ausführen eines solchen Eingriffs, zur Überwachung und zur Überprüfung nach einem Ergebnis oder Teilergebnis der Planung und die Verwendung der Vorrichtung, sowie eine Vorrichtung zum Ausführen eines solchen Eingriffs nach einem Ergebnis oder Teilergebnis der Planung und die Verwendung der Vorrichtung zu integrieren.

Ein Implantat ist ein in den Körper einzusetzendes insbesondere künstliches Material bzw. Teil, das permanent oder zumindest für einen längeren Zeitraum im Körper verbleibt.

Implantate werden z.B. nach medizinischen, funktionellen oder plastischen Implantaten unterschieden. Im Gegensatz zum Implantat wird z.B. eine Exoprothese außen am Körper angebracht.

Medizinische Implantate können z.B. Herzschrittmacher, Stents und Gefäßprothesen, Kunstherzen oder Portkatheter sein. Es sind darüber hinaus Cochlea-Implantate sowie Retina-Implantate bekannt.

In der Zahnmedizin ersetzen Zahnimplantate mit Befestigungsankern die natürlichen Zähne.

Des Weiteren sind Implantate bekannt, die dazu dienen, ein Depot eines Arzneistoffs zu bilden. Nach Verbrauch des Arzneistoffs werden die Implantate ersetzt.

Eine weitere wichtige Gruppe sind Implantate, die z.B. beschädigte Gelenke ersetzen. Die Unfallchirurgie setzt Implantate zur operativen Behandlung von Knochenbrüchen ein. Bei Schädeldefekten nach einer Trepanation oder nach einer Resektion werden Implantate aus Titan zur individuellen Schädelrekonstruktion als Knochenersatz eingesetzt.

Funktionelle Implantate dienen z.B. der Überwachung von Tieren oder Menschen. Hierbei können RFID-Chips unter der Haut des Patienten eingepflanzt werden.

Plastische Implantate werden in der plastischen Chirurgie z.B. als Ersatz für zerstörte Körperteile oder auch zur Vergrößerung von vorhandenen Körperteilen verwendet.

Heutzutage werden die meisten operativen Eingriffe zur Einbringung eines Implantats durch ein rechnergestütztes Verfahren zum Abgleich von Patientendaten, insbesondere Patientenbildern, und möglichen Implantaten geplant, beispielsweise in Form von digitalen Bildern, die unter Verwendung einer Computer-Modellerstellungs-Software interaktiv erzeugt und geprüft werden können.

Patientenbilder sind unter anderem Röntgenaufnahmen, bei denen es sich um 2D-Aufnahmen bzw. -Bilder handelt, sowie volumenbasierte Aufnahmen, wie beispielsweise CT oder MRT, bei denen es sich um 3D-Aufnahmen bzw. -Bilder handelt.

Volumenbasierte Aufnahmen, also 3D-Aufnahmen bzw. Bilder, bestehen in der Regel aus mehreren Schichten von 2D-Bildern (sog. 2D-Schichten). Diese können in einzelne 2D-Bildschichten unterteilt werden.

Volumenbasierte Bilder werden im Gegensatz zu 2D-Aufnahmen bzw. Bildern bereits bei der Aufnahme skaliert. Dies bedeutet, dass Größe des aufgenommenen Gegenstands und Maßstab der Aufnahme bekannt sind. Die jeweiligen Datenangaben zur Skalierung des Bildes werden gemeinsam mit der jeweiligen volumenbasierten Aufnahme als Datensatz gespeichert.

Demgegenüber müssen 2D-Bilder in der Regel von dem Planer/Operateur zur Planung eines operativen Eingriffs im Vorfeld der Planung skaliert werden, um Maßstab und mögliche Verzerrungen in der Tiefe des dargestellten Abschnitts eines Patientenkörpers festlegen zu können.

Gegenstand einer präoperativen Planung können beispielsweise operative Eingriffe wie Knochenresektionen, Einsetzen von Implantaten sowie Auswahl von deren Größe und Typ und verschiedene geometrische Erfordernisse, einschließlich relevanter Bemessungen, wie zum Beispiel Höhe, Breite, Umfang, Ausrichtung bestimmter Skelettteile, Knochen, Weichteile, Gewebe und Organen sein.

Bei einer chirurgischen Versorgung durch Einbringen eines Implantats werden bei der präoperativen Planung 2D-Patientenbilder, in der Regel Röntgenbildgebung, gegenüber 3D-Patientenbildern bevorzugt, da der Patient mit einer geringeren Strahlendosis belastet wird und ein 2D-Patientenbild bzw. das entsprechende Aufnahmesystem einfacher zu handhaben ist. Mit einem 2D-Patientenbild ist jedoch unter anderem in der Skalierung sowie in der räumlichen Tiefe in Blickrichtung der Aufnahme, also beispielsweise in Projektionsrichtung einer Röntgenanlage, eine Unsicherheit verbunden. Dies wirkt sich beispielsweise dahin gehend aus, dass es schwer zu klären ist, ob ein Implantat beispielsweise richtig an einem Knochen des Patienten anliegt oder anliegen würde. Weitere Unsicherheiten bestehen dahin gehend, ob Befestigungsmittel für das Implantat, z. B. Schrauben, richtig in einem Knochen greifen oder ob sie über einen Knochen hinausstehen oder in angrenzende Knochen und/oder anliegendes Gewebe und/oder Weichteile eindringen oder eindringen würden.

Planungsdaten können hier in Form der 2D-Bilddaten vorliegen bzw. aus diesen z. B. durch Bildbetrachtung oder -vermessung gewonnen werden.

Bekannte rechnergestützte Systeme zur präoperativen Planung können dem Planer/Operateur zusätzlich zur Darstellung von Patientenbildern und von möglichen Implantaten auch eine Auswahl eines bestimmten Implantats und dazugehöriger Instrumente, die bei dem chirurgischen Eingriff zu verwenden sind, vorschlagen. Gleiches gilt für Gattungen derartiger Implantate oder Instrumente.

Solcher Art Verfahren sind beispielsweise in US 2005 0228 250 A1, WO 2012/113030 A1, US 2012 0 209 394 A1, US 2012 0 027 261 A1 und US 2009 0 299 477 A1 vorgeschlagen. Das Dokument WO 2012/135653 A1 wird in diesem Zusammenhang als nächstliegender Stand der Technik betrachtet. US 2014/282008 A1 offenbart ein System zur Integration holographische Bilddaten mit Online Konnektivität.

Es ist bekannt, zum Beispiel eine individuell angepasste oder patientenspezifische Lager-Gelenkverbindung eines Kniegelenks auf der Basis des Bewegungsprofils und der Weichgewebe-/Bänderinformationen auszulegen, die für einen bestimmten Patienten zur Verfügung stehen. Ferner können Bewegungsinformationen für den Patienten durch eine reale Ganganalyse des Patienten oder durch eine Computer-Modellerstellungs-Software erhalten werden, die die 3D-Patientenbilder der Gelenke des Patienten und der dazugehörigen Bänder, Muskel- und anderen Weichgewebe zum Ableiten einer Bewegungsanalyse des Patienten und entsprechender Empfehlungen für eine Weichgewebe-Modifizierung, wie zum Beispiel das Lösen eines Bandes, verwendet.

Die Beurteilung der Operations-Situation und der Anpassung von Implantaten an den individuellen Patienten hängt wegen der oben erläuterten Nachteile der 2D-Patientenbilder jedoch sehr stark von den Erfahrungen des Operateurs und seiner Fähigkeit zur räumlichen Bildinterpretation der 2D-Bilddaten ab.

Bekannte rechnergestützte Systeme zur präoperativen Planung ermöglichen beispielsweise auch, dreidimensionale Bilder der Anatomie eines Patienten auf einer Computeranzeige oder einem anderen elektronischen Bildschirm zu betrachten, diese als Hardcopy auf einem Film oder einem anderen Medium zu reproduzieren und durch direkte oder indirekte Beleuchtung oder Hintergrundbeleuchtung zu betrachten. Ein Bild kann für eine Betrachtung auf jede geeignete Bildschirmgröße bemessen sein und kann beschnitten, gedreht etc. werden, wie von der Person (z. B. Planer/Operateur), die den Bildschirm anschaut, gewählt.

Während des Planungsprozesses kann simuliert werden, zu der betroffenen Anatomie gehöriges Weichgewebe zu modifizieren oder zu entfernen oder zu reparieren, um zum Beispiel die Ausrichtung eines Gelenks wiederherzustellen oder gerissenes oder krankes Gewebe zu entfernen oder Bänder zu schneiden oder zu reparieren oder natürliche oder künstliche Bändertransplantate vorzusehen. Informationen über Weichgewebe können wahlweise als zusätzliche Auslegungsparameter für die Auswahl eines geeigneten Implantats verwendet werden.

Aus der DE 11 2011 100 810 T5 sind ein Verfahren und eine zugehörige Einrichtung zum Herstellen eines Implantats bekannt. Hierbei wird ein dreidimensionales Bild eines Gelenks des Patienten erstellt und ein Implantat ausgewählt, das nicht individuell an das Gelenk des Patienten angepasst ist. Das Implantat wird nunmehr derart modifiziert, dass es patientenspezifisch ist. Hierbei werden mindestens sechs Winkel des Implantats modifiziert. Dabei werden anatomische und medizinische Informationen eines Patienten unter interaktiver Beteiligung eines Chirurgen integriert, um ein Implantat und wahlweise damit in Zusammenhang stehende chirurgische Instrumente für einen bestimmten Patienten aus im Wesentlichen drei Optionen auszuwählen und herzustellen: Sämtliche Implantatkomponenten, Ausrichtungsführungen und anderen Einweg-Instrumente können in einem Paket enthalten sein, das einem Chirurgen für einen spezifischen Patienten zur Verfügung gestellt wird.

Bei der bekannten Verwendung nur von 2D-Patientenbildern oder nur von 3D-Patientenbildern zur Planung eines operativen Eingriffs besteht jedoch der Nachteil, dass beispielsweise Verschiebungen von Gelenken, Belastungen, Entlastungen oder Verformungen von Knochen und Gelenken, insbesondere pathologische Verformungen der Wirbelsäule wie beispielsweise Kyphose oder Lordose, oder unterschiedliche Belastungsprofile nicht in Aufnahmesystemen abbildbar und deshalb nicht in die präoperative Planung einbeziehbar sind.

So werden beispielsweise Volumenpatientenbilder beim liegenden Patienten aufgenommen; hier erfolgt also eine Aufnahme des Patienten häufig in einem entlasteten Zustand.

2D-Patientenbilder, z.B. Röntgenaufnahmen, der Wirbelsäule, werden hingegen häufig bei stehendem Patienten aufgenommen, weil die Aufnahmesysteme diese Lage einfacher darstellen können und der betreffende Körperteil in seinem tatsächlichen Belastungszustand wiedergegeben werden kann.

Insbesondere bei der Planung von Eingriffen an der menschlichen oder tierischen Wirbelsäule, aber auch an deren Gelenken oder Skelettteilen, ist es jedoch notwendig, unterschiedliche Aufnahmesituationen und Lagen des Patienten, insbesondere stehende und liegende Situationen, beispielsweise zur Bestimmung der sagittalen Balance, einbeziehen zu können, zumal der operative Eingriff am liegenden Patienten vorgenommen wird.

Erforderlich ist es deshalb, die sich aus 2D- und 3D-Patientenbildern ergebenden Informationen - insbesondere was den Belastungs- und Entlastungszustand von Skelettteilen, Knochen, Gelenken, Wirbeln, Gewebe, Weichteilen anbelangt - dem Planer des operativen Eingriffs/Operateur auf einfache Weise zur Verfügung zu stellen, so dass er sie vorzugsweise gleichzeitig betrachten und weiterverarbeiten kann.

Aufgabe der vorliegenden Erfindung ist es, ein verbessertes Verfahren zur präoperativen Planung, zur Positionierung und/oder Ausführung eines Implantats im menschlichen oder tierischen Körper vorzuschlagen, mit dem zusätzlich zur präoperativen Planung die Überwachung des Operationsvorganges und/oder eine Prüfung nach Abschluss einer Operation möglich ist, mit dem eine gleichzeitige Erfassung, Bearbeitung und Darstellung mehrerer mehrdimensionaler Patientenbilder oder Messgrößen möglich ist, mit dem eine Weitergabe der Planungsdaten an externe Geräte und Einrichtungen möglich ist, und das vor allem Daten zur Steuerung eines externen Navigationssystem zur Verfügung stellen kann. Mit dem verbesserten Verfahren ist ein Vergleich des Planungsergebnisses mit den einzelnen Schritten der auszuführenden Operation und des Zustandes des Patienten nach der Operation möglich.

Die Aufgabe wird gelöst für das erfindungsgemäße Verfahren mit den Merkmalen der unabhängigen Patentansprüche 1, 2 oder 3, für die zur Durchführung des Verfahrens eingesetzte Vorrichtung nach den Merkmalen des Patentanspruches 38 und für die erfindungsgemäße Verwendung der Vorrichtung mit den Merkmalen des Patentanspruches 42. Nachgeordnete Patentansprüche betreffen besondere Ausführungsformen der oben aufgeführten Patentansprüche.

In der nachfolgenden Beschreibung, den Ausführungsbeispielen, den Patentansprüchen und der Zusammenfassung werden die nachstehend aufgeführten Einrichtungen und/oder Sachverhalte in folgendem Sinn verstanden:
Bildassistenzeinrichtung - ist eine bevorzugt mit elektronischen Mitteln und zugehöriger Software arbeitende Einrichtung, die eine Schnittstelle hat und es ermöglicht, Bilddaten, anatomische Daten und geometrische Daten bei Erfordernis von Datenspeichern, mit bildgebenden Verfahren arbeitenden, nicht an die Einrichtung angebundenen Geräten oder Einrichtungen oder durch Handeingabe zu empfangen, zu verarbeiten und in einem softwaregebundenen Algorithmus so aufzubereiten, dass eine präoperative Planung und/oder eine Ausgabe von Planungsdaten an interoperativ benutzte Anzeige-, Hilfs-, Mess- oder bildgebende Aufnahmeeinrichtungen übermittelt, von diesen interoperativ abruft oder abruft und in Echtzeit verarbeitet und an die angeschlossenen Einrichtungen zurück sendet.
Bildgebendes Verfahren - ist jedes prä- oder interoperativ genutzte Verfahren, durch das Informationen über den Aufbau eines menschlichen oder tierischen Körpers oder Teilen davon oder dessen Eigenschaft ermöglicht und dessen aufgenommene Bilder zum Zweck präoperativer Planung und/oder interoperativer Kontrolle durch die Bildassistenzeinrichtung übernommen, ausgewertet, verarbeitet und über eine Schnittstelle an angeschlossene Anzeige-, Hilfs-, Mess- oder bildgebende Einrichtungen übermittelt werden kann.
Schnittstelle - ist eine Einrichtung, die unter Anwendung elektrischer, funktechnischer, lichttechnischer oder magnetischer Übertragungsverfahren auch unter Einbeziehung von programmtechnischen Algorithmen in der Lage ist, von der Bildassistenzeinrichtung generierte Daten an Anzeige-, Hilfs-, Mess-, bildgebende Aufnahme- oder Navigationseinrichtungen zu übermitteln und/oder Informationen von Anzeige-, Hilfs-, Mess- oder bildgebenden Aufnahmeeinrichtungen zu empfangen.
Skizze - ist eine Darstellung des operationsrelevanten Abschnitts eines Patientenkörpers, wie sie etwa in allgemeinen biologischen oder anatomischen Darstellungen verwendet wird. Skizzen in diesem Sinne dienen insbesondere als Hilfsmittel zur besseren Visualisierung, Lokalisierung etc. des dargestellten Abschnitts des Körpers in 2D- und/oder SD-Patientenbildern. Skizzen, insbesondere Wirbelsäulenskizzen, können insbesondere den operationsrelevanten Abschnitt des Patientenkörpers, insbesondere Skelettteil, Knochen, Gelenke, Gewebe, Weichteile etc. insbesondere in seitlicher Darstellung, insbesondere als 3D-Modell, anzeigen. Diese Skizzen können unter anderem Soll-, Ideal- und Normwerte der menschlichen oder tierischen Anatomie beinhalten und mit Hilfe der Bildassistenzvorrichtung einen Vergleich der Werte des individuellen Patienten mit Norm-, Ideal- oder Sollwerten herstellen.
Das Navigationssystem - ist eine Einrichtung zur Unterstützung von Operationen. Sie dient bevorzugt der Einstellung erforderlicher geometrischer und räumlicher Verhältnisse. Sie kann die tatsächlichen geometrischen Verhältnisse erfassen und ist in der Lage, über die Schnittstelle der Bildassistenzeinrichtung sowohl Informationen von dieser zu empfangen als auch an diese zu liefern.
Steuerung - ist das über das präoperative Planungsverfahren hinausgehende Abrufen von durch die Bildassistenzeinrichtung generierten Bildinformationen durch Handeingabe, Gesten oder Informationen angeschlossener bildgebender Einrichtungen oder Informationen angeschlossener Einrichtungen zur Erfassung geometrischer Daten.

Patientenbilder sind beispielsweise durch bildgebende Verfahren entstandene zwei- oder dreidimensionale Aufnahmen, die durch bekannte Verfahren zu erhalten sind. Bevorzugt handelt es sich um 2D-Aufnahmen aus der Anwendung von Röntgenverfahren beziehungsweise um 3D Aufnahmen aus der Anwendung von CT oder MRT. Soweit im Folgenden von 2D- und/oder 3D-Patientenbildern die Rede ist, wird hierunter neben der eigentlichen Abbildung auch der zugrunde liegende Datensatz verstanden.

Zunächst ist es ein Ziel der vorliegenden Erfindung, die den 2D-Patientenbildern oder den 3D-Patientenbildern jeweils zu Grunde liegenden Informationen so zusammenzuführen oder zusammen berücksichtigen zu können, dass sie für die Visualisierung und/oder Bearbeitung des jeweils anderen Patientenbildes zur Verfügung stehen; insbesondere soll es möglich sein, die sich aus dem unbelasteten Zustand des Körpers in einem 3D-Patientenbild ergebenden Informationen für die Visualisierung und/oder Bearbeitung des 2D-Patientenbildes übertragen zu können, also die Informationen und Darstellung des 2D-Patientenbildes durch die Informationen und Darstellung des 3D-Patientenbildes zu ergänzen, zu ändern oder sonst wie zu modifizieren, bzw. umgekehrt.

Weiterhin ist es ein Ziel der Erfindung, eine Skalierung der Informationen bei den Patientenbildern vornehmen zu können.

Soweit nicht anders ausgeführt, werden die Begriffe Information und Daten im Folgenden synonym verwendet.

Der Vorteil des erfindungsgemäßen Verfahrens liegt darin, durch die mögliche gleichzeitige Darstellung und Bearbeitung mehrerer mehrdimensionaler Patientenbilder Maßnahmen der Planung, insbesondere der Simulation, eines operativen Eingriffs an unterschiedliche Anatomiemodalitäten des Patienten in unterschiedlichen Aufnahmen, insbesondere 2D-Patientenbildern und 3D-Patientenbildern, anzupassen. Ein weiterer Vorteil liegt darin, dass das Verfahren ungünstige biomechanische Ergebnisse vermeiden und durch eine optimierte Implantationsgeometrie ersetzen kann.

Erfindungsgemäß kann die präoperative Situation unter anderem durch eine automatische, halbautomatische oder manuelle Berechnung aller relevanten Winkel und sonstiger anatomischer oder operativer Parameter, etwa der Materialbeschaffenheit, der Größe etc. eines möglichen einzusetzenden Implantats, und der Patientendaten analysiert und dem Verfahren zu Grunde gelegt werden.

Das Verfahren zur Planung, Vorbereitung, Überwachung und Begleitung eines operativen Eingriffs wird an einer Bildassistenzeinrichtung durchgeführt, die dem Planer/Operateur eine archivierbare, speicherbare und reproduzierbare präoperative Planung ermöglicht und hierdurch seine Arbeit erleichtert.

Die Bildassistenzeinrichtung im Sinne dieser Erfindung ist in der Lage, dem Planer/Operateur zugängliche Datenbestände zu visualisieren, zu skalieren und zu lesen, mindestens zwei mehrdimensionale Darstellungen zumindest eines Abschnitts des Patientenkörpers zu zeigen, von denen sich wenigstens zwei Darstellungen wenigstens in Bezug auf die Anzahl der Dimensionen unterscheiden.

Ein Datenträger in diesem Sinne kann integraler oder externer Bestandteil der Bildassistenzeinrichtung sein, wie zum Beispiel der Speicher eines Personal Computers, eines Notebooks, eines Tabletcomputers, eines Smartphone, eines Speichersticks, eines optischen Datenträgers, eines Cloud-Systems oder sonstige mobile und/oder feststehende beschreibbare Speichermedien.

Erfindungsgemäß kann der Planer/Operateur der Bildassistenzeinrichtung gleichzeitig oder nacheinander mehrere auswählbare mehrdimensionale Patientenbilder, insbesondere in Form von 2D-Patientenbildern und 3D-Patientenbildern, zugänglich machen, abrufen, einspielen bzw. herunterladen und zeigen.

Die Bildassistenzeinrichtung erkennt die zugänglich gemachten Patientenbilder unter anderem in Bezug auf ihr Format z.B. als 3D-Bild und/oder 3D-Bilddaten bzw. als 2D-Bild und/oder 2D-Bilddaten.

Die Patientenbilder und/oder weitere Patienteninformationen können beispielsweise in einem PACS-System (Bildarchivierungs- und Kommunikationssystem) oder einem anderen Datenspeicher, enthalten sein. Sie können in elektronischer und/oder digitaler Form abgespeichert sein, wie beispielsweise auf einem magnetischen Speichermedium, einem optischen Speichermedium oder auf einem Halbleiterspeicher, einem sog. Cloud-System. Sie können darüber hinaus auf einer mobilen oder feststehenden Speichervorrichtung gespeichert werden.

Die Patientenbilder und Patienteninformationen können beispielsweise zu verschiedenen Zeitpunkten entstanden sein, 2D- und/oder 3D-Bild-Datensätze des Patienten und/oder, weitere Daten umfassen, insbesondere zu Körpergröße, Gewicht, Alter des Patienten, und etwa Daten aus vorangegangenen Eingriffen, Daten aus vorherigen Untersuchungen und/oder solche Daten umfassen, die in unmittelbaren Zusammenhang mit einer Operation erstellt wurden.

Die Informationen können der Bildassistenzeinrichtung beispielsweise elektronisch, über das Internet, unter Verwendung geeigneter Übertragungsprotokolle und/oder auf anderem Wege, insbesondere für den Abruf, zum Einspielen bzw. Herunterladen und Abbilden, zugänglich gemacht werden.

Die Datenübertragung kann direkt von der Festplatte eines Computers erfolgen oder mittels eines anderen mobilen oder feststehenden Datenträgers einer Festplatte, einer CD, einer DVD, einer Flash-Speichervorrichtung (z. B. Speicherstift, Compact-Flash, sichere Digitalkarte), einem sog. Cloud-System.

Ferner sind verschlüsselte oder unverschlüsselte Übermittlungen per E-Mail oder über andere digitale Übermittlungen zu jedem geeigneten Typ von Computervorrichtung, Smartphone, PDA, Tabletcomputer oder zu anderen Vorrichtungen möglich, über die elektronische Informationen übermittelt werden können.

Es ist auch denkbar, der Bildassistenzeinrichtung aus einem 3D-Patientenbild gewonnene 2D-Schichten oder bereits vorhandene externe 2D-Schichten von 3D-Patientenbildern zugänglich zu machen und für die weitere Bearbeitung und/oder Planung des operativen Eingriffs sowie des Einsatzes eines orthopädischen Implantats zur Verfügung zu stellen. Gleiches gilt entsprechend für aus 2D-Patientenbildern gewonnene 3D-Bilder.

Die Bildassistenzeinrichtung kann zudem 2D-Schichten aus einem 3D-Patientenbild ableiten und errechnen und umgekehrt. Zur Darstellung der 2D-Schicht wird in den dreidimensionalen Körper, beispielsweise Knochen, Gewebe, Gelenke oder Weichteile, insbesondere den Wirbeln der Wirbelsäule, eine Ebene gelegt, die horizontal oder vertikal oder in einem Winkel zur Achse der Wirbelsäule verlaufen kann. Die Koordinaten der Ebene bestimmen die Koordinaten der 2D-Schicht und ermöglichen es, die Perspektive einer bestimmten zweidimensionalen Schicht des dreidimensionalen Körpers darzustellen. Die Berechnung der 2D-Daten kann automatisch erfolgen.

Ferner sind die 2D- und die 3D-Patientenbilder, die dem Verfahren zur Verfügung stehen, mit Hilfe der Bildassistenzeinrichtung in einer hochauflösenden MIP- bzw. einer MPR-Darstellung anzeigbar.

Darüber hinaus können der Bildassistenzeinrichtung zur Durchführung des erfindungsgemäßen Verfahrens weitere Informationen, wie z.B. Patientenbilder, Bemaßungsdaten, Funktionsdaten und/oder sonstige Referenzdaten, Soll-Werte, Standard-Werte und/oder allgemeine patientenunspezifische Daten und/oder Informationen zu Implantaten, wie beispielsweise 2D- und 3D-Modelle von Implantaten, Herstellerinformationen, Artikelnummer, Bemaßung, Material, Verwendungsanweisungen etc., der Implantate, und/oder Skizzen sowie Norm-, Ideal- oder Sollwerte der menschlichen oder tierische Anatomie zugänglich gemacht werden.

Die vorgenannten Daten und Informationen können beispielsweise in einem Personal Computer, einem Notebook, einem Tabletcomputer, einem Smartphone, einem Daten-Stick, einer CD, einer DVD, einem Cloud-System, einem Datenarchiv oder einem sonstigen mobilen und/oder feststehenden bearbeitbaren Speichermedium gespeichert sein und der Bildassistenzeinrichtung in der oben dargestellten Weise verfügbar gemacht werden.

Patientenspezifische und allgemeine Informationen können insbesondere mit den mehreren mehrdimensionalen Patientenbildern dargestellt, abgebildet und/oder abgespeichert werden.

Ferner können erfindungsgemäß mit Hilfe der Bildassistenzeinrichtung 2D-Patientenbilder vor während oder nach deren Zugänglichmachung bearbeitet, insbesondere skaliert werden. Dabei werden automatisch, halb-automatisch oder manuell Abmessungen des abgebildeten Körperabschnitts des Patienten, insbesondere der Wirbelsäule, und/oder der Maßstab der Aufnahme mit Hilfe der Bildassistenzeinrichtung ermittelt, beispielsweise durch Verwenden eines bekannten Referenzobjekts, etwa einer Kugel aus einem nicht strahlendurchlässigen Material.

Der Skalierungsvorgang ist für jedes einzelne überspielte 2D-Patientenbild gesondert durchführbar und wiederholbar.

Die Bildassistenzeinrichtung ermöglicht es dem Planer/Operateur ferner, die zugänglich gemachten 3D-Patientenbilder in unterschiedlichen Ansichten, Ausschnitten und/oder Blickwinkeln zu segmentieren oder zu skalieren. Dies ermöglicht die Volumenerkennung und nicht nur die Erkennung einzelner Punkte der Patientenbilder. Die Segmentierung kann auch in einem Netz oder in einem Cloud-System erfolgen und gespeichert werden.

Die Segmentierung ermöglicht die Erkennung und medizinische/anatomische Bezeichnung und/oder Bezifferung einzelner Knochen, Wirbel, Gelenke, Weichteile, Gewebe etc. Die Segmentierung ermöglicht weiter die Erkennung und die medizinische/anatomische Bezeichnung und/oder die Bezifferung insbesondere der Wirbel der Wirbelsäule, anhand von Referenzmodellen und Soll-Werten. Sie ermöglicht den Vergleich mit medizinischen und anatomischen Ideal- und Normwerten der menschlichen oder tierischen Anatomie, um die durch die Operation angestrebte Patientenanatomie möglichst ideal planen zu können, die der Bildassistenzeinrichtung wie oben dargestellt zugänglich gemacht werden können. Zudem können mit Hilfe der Bildassistenzeinrichtung einzelne Knochen, Weichteile und Gewebeteile in den Darstellungen ein- oder ausgeblendet oder hervorgehoben werden.

Die Segmentierung mit Hilfe der Bildassistenzeinrichtung, also Erkennung und Bezeichnung insbesondere einzelner Knochen und/oder Skelettteile, Wirbel, Gelenke, Weichteile, Gewebe etc. kann insbesondere automatisch erfolgen und beispielsweise manuell überprüft werden.

Dies kann insbesondere bei Wirbeln, anhand von zugänglich gemachten Skizzen, insbesondere Wirbelskizzen, dargestellt, verdeutlicht und/oder überprüft werden.

Erfindungsgemäß können mit Hilfe der Bildassistenzeinrichtung an einem Bildschirm Bildschirmfenster mit unterschiedlichen Darstellungen, beispielsweise unterschiedliche Patientenbilder, Skizzen des operationsrelevanten Körperabschnitts, z.B. einer Wirbelsäule oder eines Wirbels, zeitgleich dargestellt werden.

Die Bildassistenzeinrichtung steuert den Aufbau des Bildschirms in der Form, dass der Bildschirm entweder ein einzelnes Bildschirmfenster aufweist oder in mehrere Bildschirmfenster unterteilbar ist. Der Bildschirm kann horizontal, vertikal, diagonal oder in jeder anderen Form, insbesondere zeitgleich in wenigstens zwei gleich oder unterschiedlich große Bildschirmfenster unterteilt werden.

In dem wenigstens einen Bildschirmfenster kann jeweils eine Darstellung gezeigt werden. Die Darstellungen können insbesondere Patientenbilder, Skizzen, insbesondere Wirbelsäulenskizzen, Befundungsmonitore, sonstige Abbildungen oder Daten, beispielsweise zu Implantaten umfassen. Darüber hinaus können Patientenbilder eines größeren Körperabschnitts aber auch einzelne Knochen, Gelenke, Weichteile und/oder Gewebe, insbesondere einzelne Wirbel, in einem Bildschirmfenster dargestellt werden.

Die Darstellungen wenigstens zweier Bildschirmfenster können zeitgleich gezeigt werden.

Die Darstellungen wenigstens zweier Bildschirmfenster können sich insbesondere in Bezug auf die Anzahl der Dimensionen der Darstellungen unterscheiden.

Bei dem erfindungsgemäßen Verfahren können in einer Bildassistenzeinrichtung insbesondere in wenigstens einem Bildschirmfenster zeitgleich wenigstens zwei mehrdimensionale Darstellungen zumindest eines Abschnitts des Patientenkörpers dargestellt werden, wobei operationsrelevante Daten zusammengeführt und miteinander kombiniert werden können.

Diese operationsrelevanten Daten können 2D- und/oder 3D-Bild-Datensätze des Patienten und/oder weitere Daten, insbesondere zu Körpergröße, Gewicht, Alter des Patienten, vorangegangenen Eingriffen, sowie Daten aus vorherigen Untersuchungen umfassen und/oder solche Daten, die in unmittelbarem Zusammenhang mit einer Operation erstellt wurden.

Ein Aspekt der Erfindung besteht darin, dass auf Grund des erfindungsgemäßen Verfahrens zeitgleich unterschiedliche 3D-Patientenbilder und/oder 2D-Patientenbilder in Bildschirmfenstern dargestellt werden können, insbesondere wenigstens eine Darstellung eines 2D-Patientenbildes und wenigstens eine Darstellung eines 3D-Patientenbildes in wenigstens zwei Bildschirmfenstern des Bildschirms.

Dabei ist es mit einem weiteren Aspekt der Erfindung möglich, mit Hilfe der Bildassistenzeinrichtung einen räumlichen Koordinatenbezug zwischen 2D-Patientenbildern und 3D-Patientenbildern herzustellen. Insbesondere kann eine Korrelation zwischen den vorzugsweise räumlichen Informationen von Daten und Informationen in 2D-Patientenbildern und 3D-Patientenbilder hergestellt werden und umgekehrt. Auf diese Weise sind Informationen aus einer Darstellung in eine andere Darstellung übertragbar, anzeigbar und bearbeitbar.

Das erfindungsgemäße Verfahren sieht des Weiteren die Möglichkeit vor, durch den Planer/Operateur mit Hilfe der Bildassistenzeinrichtung Patientenbilder, die in wenigstens einem Bildschirmfenster dargestellt werden, zu bearbeiten.

Insbesondere kann der Planer/Operateur beispielsweise durch Anklicken etwa mit einem Cursor, eine der Darstellungen in wenigstens einem Bildschirmfenster zur Bearbeitung in einer Arbeitsdarstellung aktivieren.

Unter Cursor in diesem Sinne ist jede Anzeige-, Eingabe-, Befehlsgebe- und/oder Bearbeitungsvorrichtung zu verstehen. Beispielsweise ist darunter eine Maus, ein Trackball, eine Tastatur, die Anzeige eines Touchscreen oder eines Grafiktabletts, insbesondere mit einem Zeiger, zu verstehen.

Dabei kann mindestens eine der mehrdimensionalen Darstellungen, insbesondere wenigstens eine 2D-Darstellung oder eine 3D-Darstellung, mit Hilfe der Bildassistenzeinrichtung als Arbeitsdarstellung aktiviert werden, wobei ein räumlicher Koordinatenbezug zwischen den einzelnen Darstellungen herstellbar ist.

Insbesondere kann die Übertragung des Koordinatenbezugs automatisch von einer 2D-Darstellung auf eine 3D-Darstellung und umgekehrt erfolgen. Dies erfolgt insbesondere unabhängig von dem Format des dargestellten Patientenbildes (insbesondere 3D- oder 2D-Patientenbild), von dem Blickwinkel, von dem die jeweilige Darstellung gezeigt wird, oder davon, welchen axialen, sagittalen, koronalen oder sonstigen Schnitt des Patientenbildes die Arbeitsdarstellung und/oder weitere Bildschirmfenster zeigen.

Möglich ist es insbesondere, in einem Bildschirmfenster ein 3D-Patientenbild und im anderen Bildschirmfenster eine 2D-Schicht dieses 3D-Patientenbildes zu zeigen, wobei die 2D-Schicht insbesondere einen axialen Schnitt etwa durch die Wirbelsäule oder den Wirbel darstellt.

Es können beispielsweise neben einem 3D-Patientenbild drei 2D-Schichten dieses 3D-Patientenbildes dargestellt werden, von denen eine 2D-Schicht einen axialen Schnitt, die andere 2D-Schicht einen sagittalen und die dritte 2D-Schicht einen koronalen Schnitt durch den jeweiligen Bereich der Wirbelsäule zeigt.

Ferner kann beispielsweise in einem Bildschirmfenster eine Skizze, z.B. eine Wirbelsäulenskizze dargestellt werden. Während der Darstellung wird wenigstens ein weiteres Bildschirmfenster vergrößert und umfassender detailliert gezeigt. Ferner kann ein entsprechender Ausschnitt eines von dem Planer/Operateur etwa mit einem Cursor markierten Bereichs dieser Skizze an einem Patientenbild dargestellt werden. Zusätzlich kann das Patientenbild selbst dargestellt werden.

Der markierte Bereich kann insbesondere einen oder mehrere Wirbel der Wirbelsäule mit und ohne Bandscheiben umfassen, wobei in der Skizze die markierten Elemente, beispielsweise Wirbel und/oder Bandscheiben, farblich abgesetzt dargestellt werden können.

In der Arbeitsdarstellung können operationsrelevante Abschnitte eines Patientenkörpers aus unterschiedlichen mehrdimensionalen Patientenbildern zur Durchführung der präoperativen Planung dargestellt, vermessen und/oder bearbeitet werden.

Die Darstellung und/oder Bearbeitung von Patientenbildern also die Auswahl einer Arbeitsdarstellung, in den einzelnen Darstellungen kann mit Hilfe eines Cursors erfolgen.

Die jeweilige Arbeitsdarstellung dient insbesondere der Bearbeitung und /oder Simulation eines operativen Eingriffs durch den Planer/Operateur.

Bestimmte Bereiche von Patientenbildern, wie beispielsweise Knochen, Gelenke, Weichteile und/oder Gewebe können insbesondere rechnergesteuert, beispielsweise mit Hilfe eines Cursors oder mit anderen Zeigevorrichtungen, mit Hilfe der wenigstens einen Arbeitsdarstellung der Bildassistenzeinrichtung in Korrelation mit einer Skizze dargestellt, vermessen und/oder bearbeitet werden. Insbesondere können einzelne Wirbel ausgewählt und markiert werden. Der ausgewählte Wirbel kann gegenüber den anderen Wirbeln farblich hervorgehoben werden.

Die Bearbeitungs- und/oder Simulationsschritte des Planers/Operateurs in einer Arbeitsdarstellung können insbesondere rechnergesteuert etwa mit Hilfe eines Cursors oder anderen Eingabemethoden erfolgen.

Durch Markieren, beispielsweise Anklicken eines Knochens, Gelenks, Weichteils oder Gewebes in der Arbeitsdarstellung, insbesondere eines oder mehrerer Wirbel der Wirbelsäule, kann der Planer/Operateur in der wenigstens einen Arbeitsdarstellung insbesondere an einem Bereich der Wirbelsäule, in einem 2D-Patientenbild und/oder in einem 3D-Patientenbild Planungsschritte durchführen und einzelne Operationsschritte simulieren.

Aufgrund des erfindungsgemäßen Koordinatenbezuges zwischen den 2D-Patientenbildern und den 3D-Patientenbildern können die jeweiligen Bearbeitungsschritte des Planers/Operateurs koordinatenbezogen in die anderen Darstellungen übernommen werden. Hierdurch sind sämtliche Bearbeitungsschritte in allen ausgewählten Darstellungen, insbesondere für alle Belastungs- und Entlastungslagen des jeweiligen Körperabschnitts und in allen ausgewählten Dimensionen und im Vergleich zu Sollwerten und Idealwerten beispielsweise aus Skizzen darstellbar, planbar und prüfbar.

Ferner kann zur präoperativen Planung, insbesondere zur Simulation eines operativen Eingriffs, in einer oder mehreren der Darstellungen des Körperabschnitts des Patienten, an dem der Eingriff erfolgt, die als Arbeitsdarstellung/en ausgebildet ist/sind wenigstens ein bewegbares und im Verhältnis zum Körperabschnitt bewegbares Modell eines Implantat angeordnet werden.

Ein wesentlicher Aspekt des erfindungsgemäßen Verfahrens besteht darin, dass die Arbeiten und Planungsschritte, die der Planer/Operateur in der Arbeitsdarstellung, insbesondere im Zusammenhang mit einem ermittelten möglichen Implantat, durchführt - beispielsweise die Positionierung des möglichen Implantats anhand eines dargestellten Implantat-Modells, insbesondere an einem Wirbel der Wirbelsäule, die Veränderung der Form und/oder der Größe des Implantat-Modells, die Verdrehung oder Verschiebung des Implantat-Modells, die Veränderung des Anstellwinkels eines Implantat-Modells beispielsweise zu einem Wirbel, und/oder Bemaßungen oder Messergebnisse - zeitgleich und in den jeweils entsprechenden räumlichen Koordinaten vorzugsweise durch Herstellung eines Koordinatenbezugs auf wenigstens einem oder auf mehreren oder auf allen weiteren Bildschirmfenstern dargestellt werden können.

Die Bildassistenzeinrichtung ermöglicht dabei insbesondere eine Rundumansicht des operationsrelevanten Abschnitts des Patientenkörpers anhand der Patientenbilder und möglicher Implantat-Modelle in der jeweiligen Arbeitsdarstellung. Dabei können insbesondere Darstellungen von Körperregionen und Implantat-Modellen etwa mit Hilfe des Cursors durch den Planer/Operateur ein- und ausgeblendet werden, fokussiert, eingesetzt oder bemessen werden.

Mit Hilfe der Bildassistenzeinrichtung werden die zugänglich gemachten Daten, insbesondere Bemaßungen, Messergebnisse etc. innerhalb eines Vergleiches der individuellen Patientendaten mit Skizzen oder Soll-, Ideal- oder Normwerten bewertet und auf Abweichungen zwischen Soll- und IstWerten untersucht, um ein optimales post-operatives Ergebnis für diesen Patienten zu erzielen.

Mit Hilfe der Bildassistenzeinrichtung, insbesondere mit Hilfe der wenigstens einen Arbeitsdarstellung, können fachspezifische Bemaßungen, wie etwa Länge, Tiefe, Umfang, Höhe und Breite und/oder Dichte, beispielsweise eines Knochens und/oder Skelettteils, eines Gewebes, eines Weichteils oder Organs, in Form von Referenzpunkten dargestellt werden anhand derer Referenzpunkte an Implantat-Modellen, die in einer Datenbank gespeichert sein können, abgeglichen werden und so automatisch oder manuell passende Implantate, beispielsweise Cages (Implantat-Käfige), Bauteile, künstliche Gelenke, Schrauben, Platten, Fixateure, Implantatkomponenten, Ausrichtungsführungen, Einweg-Instrumente, Befestigungsmittel, Pfanne und Schaft für ein Hüftimplantat etc., für den jeweiligen Patienten automatisch ausgewählt und vorgeschlagen werden.

In einem vorzugsweise automatischen Auswahlverfahren zur Planung eines operativen Eingriffs werden anhand des Abgleichs von bekannten und/oder ermittelten Referenzpunkten zwischen Implantat-Informationen und den Patientenbildern wenigstens ein am besten passendes Implantat ermittelt, vorgeschlagen und automatisch mit Hilfe der Bildassistenzeinrichtung in eine der Darstellungen, z.B. in das 3D-Patientenbild, vorzugsweise in die wenigstens eine Arbeitsdarstellung eingeblendet.

Erfindungsgemäß wird zum Zweck der genauen Auswahl und/oder Einpassung eines möglichen Implantats beispielsweise die innere Kortikalis des Patienten auf den Patientenbildern mit Hilfe der Bildassistenzeinrichtung automatisch erkannt. Dabei kann mit Hilfe der Bildassistenzeinrichtung durch optische, insbesondere farbliche, Markierungen dargestellt werden, ob sich Messergebnisse innerhalb- oder außerhalb eines festgelegten Normbereichs befinden.

Der Planer/Operateur kann darüber hinaus nach eigenen Entscheidungskriterien manuelle Änderungen bei der Auswahl des Implantats vornehmen.

Insbesondere ist es dem Planer/Operateur in der wenigstens einen Arbeitsdarstellung möglich, bei der Planung und/oder Simulation eines operativen Eingriffs an der Wirbelsäule durch Markieren, insbesondere Anklicken mit dem Cursor, wenigstens ein Teil beispielsweise eines Wirbels auf einem Patientenbild zu entfernen. Ferner ist es möglich, durch Markieren, insbesondere Anklicken, etwa mit einem Cursor andere Teile am Wirbel anzuordnen oder z.B. ein Implantat am Wirbel zu befestigen. Je nach der benötigten Perspektive kann in einer Arbeitsdarstellung eine 3D-Darstellung mit Hilfe etwa eines Cursors durch den Planer/Operateur gedreht, geschwenkt und/oder geneigt werden.

Es ist insbesondere auch vorstellbar, Schnitte durch einen beliebigen dargestellten Körperteil, etwa durch Wirbel, und weitere mögliche Schritte der zu planenden und durchzuführenden Operation zu simulieren, um nachvollziehen zu können, welche Auswirkung eine bestimmte Anordnung eines Implantats oder ein bestimmter Operationsschritt in Bezug auf den inneren Bereich des Körperteils hat.

Eine mit der 3D-Darstellung korrespondierende 2D-Darstellung kann ebenfalls mittels des Cursors bearbeitet werden. Dabei können 2D-Darstellungen und/oder 2D-Schichten z.B. mit Hilfe des Scrollrades einer Maus oder eines ähnlichen rechnerverbundenen Zeigemechanismus durchlaufen werden. Die jeweilige Arbeitsposition wird zeitgleich und in den entsprechenden räumlichen Koordinaten auf der jeweiligen Darstellung in jedes andere Bildschirmfenster übertragen. Das erfindungsgemäße Verfahren ermöglicht so eine genaue Orientierung und Übertragung von Koordinaten sowohl in 2D-Darstellungen als auch in korrespondierenden 3D-Darstellungen eines dargestellten Abschnitts des Patientenkörpers. Der Cursor verdeutlicht, welchen Bereich einer 3D-Darstellung und/oder einer 2D-Darstellung der Planer/Operateur zum jeweiligen Zeitpunkt markiert und/oder beplant und/oder bearbeitet.

Derartige Bearbeitungsmaßnahmen etwa in 3D-Patientenbildern können vorzugsweise automatisch in ihren Auswirkungen auch in 2D-Patientenbildern, vorzugsweise unter Herstellung eines Koordinatenbezuges, wiedergegeben werden. Gleiches gilt umgekehrt, wenn die Bearbeitungsmaßnahmen in 2D-Patientenbildern vorgenommen werden.

Erfindungsgemäß ist es insbesondere zur präoperativen Planung von Wirbelsäulenoperationen beispielsweise möglich, auf Basis der Patientenbilder die sagittale Balance rechnergestützt zu vermessen. Hierzu werden auf wenigstens einer der Darstellungen in den Bildschirmfenstern von dem Planer/Operateur Bestimmungspunkte mit Hilfe des Cursors markiert. Dies sind insbesondere der ventrale Endpunkt der Sakrumbasis, der ventral-craniale Endpunkt der Sakrumbasis und/oder die beiden Femurkopf-Mittelpunkte. Die Messergebnisse können rechnergestützt darüber hinaus einem Soll-/IstVergleich mit Soll-, Ideal- und Normwerten der menschlichen oder tierischen Anatomie unterworfen werden und in einer Ergebnisliste aufgeführt und bewertet werden. Auf diese Weise kann insbesondere ein mögliches einzusetzendes Implantat nach bestimmten Parametern ausgewählt und positioniert werden.

Für die weitere Bearbeitung an der jeweiligen Position auf einem 3D- bzw. 2D-Patientenbild wird in der wenigstens einen Arbeitsdarstellung mit Hilfe des Cursors durch den Planer/Operateur ein Implantat-Modell in die 3D-bzw. 2D-Darstellung eingefügt.

Im Weiteren ist das Implantat beispielsweise eine Schraube an einem Wirbelkörper der Wirbelsäule.

Für die Auswahl der Schraube steht eine Vielzahl von möglichen Parametern zur Verfügung. Auswahlkriterien sind z.B. der gewünschte Hersteller der Schraube, der Schraubentyp, die Ausführung der Schraube, das Schraubenmaterial sowie die Größe und die Länge.

Dabei können mit dem erfindungsgemäßen Verfahren zunächst die gewünschten Parameter der Schraube mit Hilfe der Bildassistenzeinrichtung festgelegt werden. In einem nächsten Schritt erfolgt die Auswahl des operationsrelevanten Knochens, insbesondere eines Wirbelkörpers, z.B. in einer Skizze und/oder auf einem Patientenbild in einer der Darstellungen in den Bildschirmfenstern. In wenigstens einer Darstellung in einem weiteren Bildschirmfenster sind ausgewählte Ausschnitte darstellbar.

Das an Hand vorgegebener Parameter ausgewählte Implantat-Modell, beispielsweise eine Schraube, kann rechnergestützt in der Arbeitsdarstellung in einem mehrdimensionalen Patientenbild vorplatziert werden. Zeitgleich wird das Implantat-Modell in der ausgewählten Form in weiteren Darstellungen in den Bildschirmfenstern an den entsprechenden räumlichen Koordinaten des jeweils in den Darstellungen abgebildeten Patientenkörpers angezeigt. Die Positionierung der Schraube an einem Wirbel in den übrigen Bildschirmfenstern entspricht dabei der Anordnung und Ausrichtung der Schraube an einem Wirbel in der Arbeitsdarstellung.

Dabei kann in der Arbeitsdarstellung die Positionierung eines Implantat-Modells, beispielsweise einer Schraube, ohne Abänderung der Neigung, der Richtung sowie des Winkels etwa mit Hilfe des Cursors, beispielsweise durch Ziehen mit dem Cursor auf oder entlang der X-, Y- und/oder der Z-Achse, verschoben und/oder auf oder entlang von Kreisen, die jeweils die Drehachse bzw. die mögliche Drehrichtung wiedergeben, gedreht werden.

Es besteht ferner die Möglichkeit, für ein Implantat-Modell, beispielsweise ein Schraube, in der Arbeitsdarstellung eine Bohrung in einem Knochen, beispielsweise einem Wirbel, zu simulieren, wobei die Tiefe der Bohrung durch den Planer/Operateur verlängert oder verkürzt werden kann.

Es ist ferner möglich, in der Arbeitsdarstellung mit Hilfe des Cursors die Veränderung des Neigungswinkels, des Drehwinkels sowie des Schwenkwinkels des Implantat-Modells, beispielsweise eine Schraube, in einem Knochen, beispielsweise einem Wirbel, zu simulieren. Hierbei wird die Simulation auf andere benachbarte Knochen, insbesondere Wirbel übertragen unter Beibehaltung der simulierten Form und Güte sowie unter Beibehaltung der simulierten Ausrichtung gegenüber dem dargestellten Knochen. Vorzugsweise erfolgt die Simulation anhand von Wirbeln der Wirbelsäule im Vergleich zu den umliegenden Knochen und Weichteilen, beispielsweise den Wirbeln der Wirbelsäule.

Auf die dargestellte Weise können unterschiedliche Implantate und deren räumliche Anordnung am Patientenbild simuliert und im Hinblick auf ihre Auswirkung und Verträglichkeit gegenüber benachbarten Knochen, Gewebe, Weichteilen, Organen, etc. geplant werden. Der Operateur hat somit die Möglichkeit, für jeden Patienten das optimale Implantat sowie die optimale Ausrichtung des Implantats zu planen und/oder zu simulieren.

Die Erfindung erweist sich auch deshalb als vorteilhaft, da die Ergebnisse des operativen Eingriffs, insbesondere des Einsatzes wenigstens eines orthopädischen Implantats durch die zeitgleiche Darstellung in wenigstens zwei Bildschirmfenstern und aus unterschiedlichen Blickwinkeln bzw. Perspektiven der Darstellungen weiterer Bildschirmfenster kontrolliert und gegebenenfalls korrigiert werden können. Dies erfolgt unabhängig davon, ob die simulierte Handlung in einer 3D- oder einer 2D-Arbeitsdarstellung erfolgt ist oder ob wenigstens ein weiteres Bildschirmfenster neben der Arbeitsdarstellung geöffnet ist.

Ein weiterer Vorteil des erfindungsgemäßen Verfahrens besteht darin, dass bei der Vielzahl der durchführbaren Messungen zur Planung des Einsatzes des Implantats eine vollautomatische Erkennung der Knochen, Gewebe, Weichteile, Gelenke, Wirbel etc., die in den Patientenbildern dargestellt sind, und deren Bezeichnung möglich ist.

Das Verfahren ermöglicht insbesondere, zur Planung von Wirbelsäulenoperationen die Berücksichtigung von Nash-Moe-Rotation, von innerem Pedikel-Abstand, von Spinalkanal-Weitenindex, von Lordose, von Kyphose, von atlantodentale Distanzen, des Durchmesser des Wirbelkanals, von Wirbellinien, der Mc Gregor's-Linie, von Spondylolisthese, der Bandscheibenhöhe, der Bandscheibenwinkel, der Instabilität nach van Akkerveeken, des sakralen Winkels, von Funktionsanalysen, von Stabilitätskriterien bei der Spondiloretrolysthese, der Anordnung von Grund- oder Deckplatten, der Anordnung eines Lots zum Wirbelkörper-Schwerpunkt, der zervikale Schwerelinie, von Rissergrad, von Skoliose nach Cobb oder nach Ferguson, von Pelvic Tilt PT und/oder von Pelvic Incidence PI, von Pelvic Angulation PA, von Pelvic Lordosis Angle, von Pelisacral Angle PSA und/oder von Sacral Slope, von C7 Plumb Line, von Pelvic Thickness CS, von Pelvic Thickness SPT , etc., mit denen einzelne Schritte eines operativen Eingriffs an der Wirbelsäule in einer Arbeitsdarstellung simuliert werden können. Insbesondere aus der Anterior/Posterior- und der Sagittal-Ansicht der Wirbelsäule können zudem beispielsweise Apex-Winkel und tatsächlicher Winkel bestimmt und dem Verfahren zu Grunde gelegt werden.

Das Verfahren kann unter anderem auch bei der Planung von Hüftimplantaten verwendet werden. Das Verfahren erleichtert dem Operateur die Auswahl und Einpassung der geeigneten Pfannen- und Schaftkombinationen. Darüber hinaus kann mit dem erfindungsgemäßen Verfahren die Adduktion und/oder Abduktion der Gliedmaßen gegenüber der Hüfte korrigiert werden. Weiter ist es möglich, präoperativ sowie postoperativ den Beinlängenausgleich zu ermitteln und darzustellen.

Ferner kann durch das erfindungsgemäße Verfahren eine biometrische Analyse erstellt und ein Vorschlag für die optimierte Lage von Gelenkmittelpunkten erstellt werden. Körpergröße, Gewicht und die biomechanische Ausgangsanalyse des Patienten können automatisch berücksichtigt werden. Bei der Durchführung einer biometrischen Analyse kann mit dem erfindungsgemäßen Verfahren beispielsweise der Drehpunkt für eine optimierte Gelenkgeometrie simuliert werden, wobei die Belastungssituation eines gesunden Gelenkes zu Grunde gelegt werden kann. Dergleichen Bemaßungen und Messergebnisse können rechnerisch ermittelt und angezeigt werden.

Bei der Planung eines operativen Eingriffs in eine kindliche Hüfte (Coxometrie) können dem operativen Eingriff klinisch relevante Bemaßungen zur Beurteilung von Hüftgelenken zu Grunde gelegt werden. Zur Berücksichtigung national unterschiedlicher Kriterien bei der Planung eines operativen Eingriffs an der kindlichen Hüfte basiert das Verfahren auf Graduierungstabellen, die diese national verschiedenen Kriterien berücksichtigen, so dass das Verfahren international eingesetzt werden kann.

Ferner kann das erfindungsgemäße Verfahren auch zur Knie-Endoprothetik eingesetzt werden. Insbesondere können Achsfehlstellungen einer Knieprothese noch während der präoperativen Planung ermittelt und korrigiert werden.

Mit Hilfe des Verfahrens kann eine Korrektur der Fehlstellung beispielsweise des Beins abhängig von der jeweiligen Zielsetzung automatisch und/oder manuell vorgenommen werden. Hierzu werden die postoperativ zu erwartenden mechanischen Achsen, die Traglinie sowie alle relevanten Winkel ermittelt.

Erfindungsgemäß kann das Verfahren zudem zur Planung einer Osteotomie verwendet werden.

Hierzu kann die gezielte Durchtrennung einzelner oder mehrerer Knochen, beispielsweise um Fehlstellungen der Beinachsen oder der Hüftfehlstellung zu korrigieren, geplant werden. Das erfindungsgemäße Verfahren ermöglicht es weiter, eine oder mehrere Osteotomien nach der Art, der Anzahl, der Größe und der Lokalisierung festzulegen. Insbesondere femorale oder tibiale Umstellungs-Osteotomien können mit Hilfe des Verfahrens ebenso geplant werden, wie einfache oder mehrfache Osteotomien nach dem Open-Wedge-Verfahren oder nach dem Closed-Wedge-Verfahren.

Nach einer durchgeführten Korrektur können alle gelenkbezogenen, mechanischen Belastungsachsen und Gelenktangenten erfindungsgemäß einem Soll/IstVergleich der Patientendaten mit Soll-, Ideal- und Normwerten oder Skizzen der menschlichen oder tierischen Anatomie unterzogen werden, um ein möglichst optimales post-operatives Ergebnis für den Patienten zu erzielen.

Zur Planung der Osteotomie kann die Bildassistenzeinrichtung Teilbilder aus einzelnen Darstellungen und/oder Patientenbildern zu einer einzigen Darstellung, beispielsweise zur Simulation der Verbindung von Knochenenteilen, zusammenfügen. Darüber hinaus kann der Operateur mit Hilfe der Bildassistenzeinrichtung einfache Bildbearbeitungen für eine Rekonstruktion von Skelettelementen durchführen.

Ein weiterer Vorteil der Erfindung besteht darin, dass zur Planung der Osteotomie Knochensegmente freigestellt, verschoben und erforderliche Osteosynthese-Implantate platziert werden können. Fehlstellungswinkel können z.B. nach Dror Paley berechnet werden.

Zudem können zur Osteosynthese geeignete und/oder erforderliche Komponenten wie Nägel, Platten, Schrauben etc. ausgewählt werden.

Mit Hilfe des Verfahrens kann des Weiteren ein operativer Eingriff an Schultern, Ellenbogen, Händen und Fingern geplant und die Auswahl und die Position von Implantaten optimal vorbereitet werden.

Das erfindungsgemäße Verfahren kann auch eine Verbesserung der Krafteinleitung und der Wiederherstellung des physiologischen Muskelspiels an Hand von biomechanisch ermittelten Normbereichen, nach denen die Implantate verankert werden, ermöglichen.

Gleiches gilt auch für die Planung eines operativen Eingriffs am menschlichen und/oder tierischen Fuß, am Sprunggelenk oder am Zeh, beispielsweise der nach außen gerichteten Schiefstellung der Großzehe im Grundgelenk (Hallux Valgus).

Das erfindungsgemäße Verfahren ermöglicht es dem Planer/Operateur darüber hinaus, Achsfehlstellungen zu ermitteln und automatisch oder manuell zu korrigieren.

Prä- und auch postoperative Messergebnisse können verfahrensgemäß in der wenigstens einen Arbeitsdarstellung angezeigt werden.

Erfindungsgemäß können während des Planungsvorgangs neben den einzelnen Arbeitsschritten auch Kommentare und Anmerkungen zur Planung über die Bildassistenzeinrichtung gespeichert werden und verfügbar gemacht werden. Darüber hinaus können einzelne Arbeitsschritte, Kommentare oder Anzeigen in der Arbeitsdarstellung ein -und ausgeblendet werden.

Erfindungsgemäß können das endgültige Planungsergebnis eines möglichen zu planenden operativen Eingriffs und/oder Planungsschritte der Planung, also Teilergebnisse der Planung, jeweils als Datensätze kabellos, beispielsweise über Bluetooth oder sonstige mobile Übermittlungssysteme, oder kabelgebunden gespeichert werden, insbesondere in der Bildassistenzeinrichtung, aber auch in jedem anderen Speichermedium, wie beispielsweise einem externen Rechner, etc., oder in Archivsystemen.

Das endgültige Planungsergebnis, also die Darstellung des geplanten postoperativen Zustandes des Patienten, kann insbesondere in einer Navigationsansicht der Darstellung der Bildassistenzeinrichtung dargestellt werden. Dort werden anstelle der Implantat-Modelle Führungslinien angezeigt, welche die Ausrichtung und Positionierung des Implantats darstellen.

Ferner kann das endgültige Planungsergebnis eines möglichen zu planenden operativen Eingriffs und/oder Teilergebnisse der Planung beispielsweise über Bluetooth oder sonstige mobile Übermittlungssysteme, oder kabelgebunden, auch auf mobile Endgeräte, wie Smartphones, Tabletcomputer, Notebooks, übertragen, dort dargestellt, bearbeitet und/oder gespeichert werden.

Erfindungsgemäß können das endgültige Planungsergebnis eines möglichen zu planenden operativen Eingriffs und/oder Teilergebnisse der Planung über eine Schnittstelle an ein nicht zur Bildassistenzeinrichtung gehörendes Gerät übermittelt werden. Es ist möglich, Daten und/oder Befehle an externe Geräte zu übermitteln, beispielsweise an eine weitere Bildassistenzeinrichtung in einem Operationssaal.

Auf diese Weise können das endgültige Planungsergebnis eines möglichen zu planenden operativen Eingriffs und/oder Teilergebnisse der Planung im Operationssaal dargestellt werden.

Externe Geräte können Bildassistenzeinrichtungen, Anzeige-, Hilfs-, Messgeräte oder vorzugsweise ein mobiles oder ein stationäres (Operations-) Navigationssystem sein. Das Navigationssystem dient insbesondere zur Steuerung und Überwachung eines operativen Eingriffs, insbesondere zur Bestimmung der örtlichen Position und/oder zur Bestimmung der Koordinaten des Eingriffs und der Einleitung und der Durchführung von operativen Maßnahmen. Die Planungsdaten können beispielsweise kabellos oder kabelgebunden über geeignete Protokolle übermittelt werden.

Die Schnittstelle kann ferner so konfiguriert sein, dass sie in der Lage ist, entfernt angeordnete Anzeigegeräte mit Informationen zu versorgen. Beispielsweise kann dies ein in einem Operationssaal angeordneter Bildschirm sein.

Die Schnittstelle kann so ausgelegt sein, dass sie nicht nur Daten von externen Quellen übernehmen kann oder nur Steuerdaten an eine Anzeigeeinrichtung liefern kann. Bei entsprechender Ausrüstung der Schnittstelle ist es möglich, permanent Daten von externen Quellen zu laden, diese mit gespeicherten Daten zu vergleichen und über eine Anzeigeeinrichtung dem Operateur zur Verfügung zu stellen. Mögliche Daten sind dabei neben den oben beschriebenen Bilddaten auch durch externe Messeinrichtungen ermittelte geometrische Daten, beispielsweise Daten über Lage, Ausrichtung oder Fortschritt eines operativen Eingriffs.

Dabei ist es unter anderem möglich, sog. elektronische Zäune, beispielsweise zur Begrenzung des Operationsbereichs und/oder zur Markierung von bestimmten Bereichen im Körper des Patienten, zur genauen Positionierung eines Implantats in der mehrdimensionalen, vorzugsweise dreidimensionalen Planung, festzulegen und zur Ein- und/oder Ausgrenzung dieser Bereiche sowie zur örtlichen Koordination des Eingriffs und/oder des Implantats vorzunehmen und die zugrunde liegenden Koordinaten an ein mobiles oder stationäres Operations-Navigationssystem kabellos oder kabelgebunden zu überspielen.

Es ist weiterhin möglich, die bei der präoperativen Planung ermittelten Daten in ein 3D-Modell des Implantats zu übersetzen und diese Daten über die Schnittstelle an mit dieser verbundene Maschinen zu senden. Dies können beispielsweise spanabhebende Werkzeugmaschinen sein, die entweder ein neues Implantat herstellen oder vorhandene Implantate individuell nachbearbeiten. Ebenso ist es möglich, einen 3D-Drucker anzusteuern, der entweder ein angepasstes Implantat herstellt oder ein nicht als Implantat geeignetes Muster erzeugt, das abhängig vom Bedarf Studienzwecken während der präoperativen Planung dient, als Modell für Lehrzwecke geeignet ist oder auch als Modell für ein durch Urformen herzustellendes Implantat dienen kann.

Eine weitere Möglichkeit besteht darin, dass die Ergebnisse der präoperativen Planung über die Schnittstelle einem Navigationssystem übermittelt werden, wobei entweder direkt Steuerdaten übermittelt werden oder das Navigationssystem aus erhaltenen Planungs- oder Bilddaten selbst Steuerdaten gewinnt.

Es ist ebenso möglich, dass die Bildassistenzeinrichtung über seine Schnittstelle nicht nur Daten an angeschlossene Geräte liefert. Es kann ebenso über die Schnittstelle eine Rückübertragung von Daten der externen Geräte an die Bildassistenzeinrichtung durchgeführt werden. Damit wird es möglich, durch ständigen Vergleich der Soll-, Planungs- und Ist-Daten während der Operation eintretende Veränderungen festzustellen, um faktisch ein Monitoring der durchgeführten Operation zu ermöglichen.

Neben der automatischen Erfassung von Daten und dem automatischen Abgleich ist des Weiteren eine zusätzliche visuelle Überwachung der Vorgänge im zu operierenden Körperteil beziehungsweise im Körper zu erkennen, die der Operateur möglicherweise nicht erkennen kann.

Kann die Bildassistenzeinrichtung oder die Schnittstelle ein Monitoring ausführen oder ermöglichen, wird dadurch gewährleistet, über die Schnittstelle auch Daten oder Befehle an angeschlossene externe Geräte zu übermitteln. Insbesondere bei der Verwendung eines Navigationssystems besteht so die Möglichkeit, auf Abweichungen von geplanten Maßnahmen und dem Planungsergebnis aufmerksam zu machen und somit Unregelmäßigkeiten bei der Durchführung der Operation zu vermeiden. Die Art der gegebenen Hinweise kann von den Fähigkeiten des Navigationsgerätes abhängen. Es kann beispielsweise eine optische und/oder akustische Information ausgegeben werden.

Die Bildassistenzeinrichtung kann über die Schnittstelle Bilddaten an einen Monitor im Operationssaal ausgeben. In Fällen, in denen der Operateur Details der präoperativen Planung oder aktuelle Veränderungen nachvollziehen muss, kann über die Schnittstelle eine Gestensteuerung an das Bildassistenzeinrichtung angekoppelt werden. So besteht die Möglichkeit, trotz vorhandener hygienischer Bedingungen im Operationssaal zeitnah nachzuprüfen, ob die Operation mit ihren Ergebnissen den Vorgaben des Planungsergebnisses entspricht.

Nach einem vorteilhaften Aspekt der Erfindung wird das Planungsergebnis und/oder Teilergebnisse der Planung an eine Bildassistenzeinrichtung im Operationssaal oder ein Navigationssystem (47) übermittelt. Dort kann die Arbeitsdarstellung als Navigationsansicht aufgerufen werden, so dass das Planungsergebnis und/oder Teilergebnisse der Planung mit Führungslinien angezeigt werden.

Dieses Planungsergebnis kann über die Schnittstelle der Bildassistenzeinrichtung mit Patientenbildern, insbesondere 2D- und/oder 3D-Patientenbildern korreliert werden. Dabei wird eine oder mehrere Röntgen-, CT-, MRT- oder andere in einem bildgebenden Verfahren hergestellte Aufnahme des Patienten in der Lage auf dem Operationstisch erstellt.

Zusätzlich besteht die Möglichkeit, die Aufnahme mit bekannten Verfahren zu kalibrieren. Mit Hilfe der Schnittstelle der Bildassistenzeinrichtung wird eine Korrelation der Koordinaten der mehreren Patientenbilder hergestellt, so dass das Patientenbild des Patienten auf dem Operationstisch in Koordination, Ausrichtung und Darstellung mit der Darstellung des Planungsergebnisses in der Bildassistenzeinrichtung, insbesondere der Navigationsansicht übereinstimmt.

Über die Schnittstelle der Bildassistenzeinrichtung und des Navigationssystems ist es zudem möglich, das Planungsergebnis und/oder die Teile des Planungsergebnisses und/oder das mindestens auf dem Operationstisch von dem Patienten erstellte Patientenbild einer bildgebenden Vorrichtung, beispielsweise einem Röntgengerät, einem MRT-Gerät oder einem CT-Gerät zur Verfügung zu stellen. Zudem ist es über die Schnittstelle der Bildassistenzeinrichtung und des Navigationssystems möglich, der Bildassistenzeinrichtung und dem Navigationssystems Daten und Informationen zur Verfügung zu stellen, zu speichern, anzuzeigen und darzustellen.

Erfindungsgemäß kann das Planungsergebnis und/oder Teile des Planungsergebnisses und/oder Patientenbilder, die von den Patienten auf dem OP-Tisch angefertigt wurden, an eine bildüberwachende Vorrichtung kabelgebunden oder kabellos übermittelt werden. Die bildüberwachende Vorrichtung ermittelt nach bekannten Verfahren virtuell Lage und Position des Patienten sowie die Lage, die Position und den Fortschritt von einzelnen Schritten der geplanten Operation.

Auf Grund der Kalibrierung sind die tatsächlichen Abmessungen und Dimensionen des Patienten sind mittels der Bildassistenzeinrichtung gespeichert und können dem Navigationssystem über eine Schnittstelle übermittelt werden.

Instrumente oder Implantate sind beispielsweise aus der verfahrensgemäßen Planung ebenfalls mittels der Bildassistenzvorrichtung gespeichert oder werden mit Hilfe der Bildassistenzeinrichtung zugänglich gemacht, beispielsweise unter Zugriff auf bekannte gespeicherte Informationen oder durch bildgebende und kalibrierte Aufnahmen der Instrumente oder Implantate.

Die Bildassistenzeinrichtung und das Navigationssystem ermöglichen eine automatische Korrelation der Position, Ausdehnung und der Lage des Patienten mit der Position, Ausrichtung und Lage von Implantaten oder Instrumenten.

Zusätzlich können die Instrumente oder Implantate mit wenigstens einem Ortungsmittel verbunden sein, welche die relative Position der Instrumente oder Implantate und Veränderungen in Position, Ausdehnung und Lage der Instrumente oder Apparate an die Bildassistenzeinrichtung und das Navigationssystem übermitteln können.

Mit Hilfe der Bildassistenzeinrichtung und des Navigationssystem können alle Werkzeuge, Implantate und die Lage des Patienten relativ zueinander so dargestellt werden, wie sie auch tatsächlich im Raum liegen.

Insbesondere werden die Führungslinien der geplanten Eingriffe und der geplanten Implantate sowie elektronische Zäune mit Hilfe des Navigationssystem (47) oder der Bildassistenzeinrichtung angezeigt. Dem Operateur ist es somit möglich, in einem Echt-Zeit-Vergleich die einzelnen Operationsschritte an die präoperativ geplanten Operationsschritte anzupassen. Der Operateur kann erfindungsgemäß insbesondere Lage, Position, Winkel, Tiefe, Positionierung und Durchführung der präoperativ geplanten Schritte an das Planungsergebnis anpassen. Der Operateur kann hierdurch verhindern, beispielsweise zu tief und/oder in einem falschen Winkel oder an der falschen Stelle Operationsschritte vorzunehmen.

Erfindungsgemäß wird zudem eine Vorrichtung zur Ausführung eines operativen Eingriffs nach dem erfindungsgemäß erlangten Planungsergebnis eines operativen Eingriffs und/oder Teilergebnisse der Planung zur Verfügung gestellt und zur Ausführung eines erfindungsgemäß geplanten operativen Eingriffs verwendet.

Weitere Vorteile und Einzelheiten der vorliegenden Erfindung ergeben sich aus den im Folgenden beschriebenen Ausführungsbeispielen sowie anhand der Zeichnungen.

Hierbei zeigen:
- Fig. 1: einen schematisierten Ablaufplan,
- Fig. 2: einen Bildschirm mit zwei Bildschirmfenstern,
- Fig. 3: ein Bildschirmfenster mit einer Skizzendarstellung sowie ein Bildschirmfenster mit einer 3D-Darstellung,
- Fig. 4: eine Wirbelsäulenskizze mit einem Wirbelbereich und eine zugehörige 3D-Darstellung,
- Fig. 5: ein Bildschirmfenster mit einer 3D-Darstellung sowie ein Bildschirmfenster mit einer 2D-Darstellung,
- Fig. 6: einen Bildschirm mit vier Bildschirmfenstern, und
- Fig. 7: zwei Bildschirmfenster mit einem Implantat.

Im Weiteren wird das erfindungsgemäße Verfahren an Hand der Planung eines operativen Eingriffs an einem Wirbel der Wirbelsäule eines Menschen beschrieben. Dies ist jedoch in keiner Weise einschränkend oder ausschließlich gemeint, da das Verfahren zur Planung des operativen Eingriffs auch an anderen Stellen des menschlichen oder tierischen Körpers eingesetzt werden kann.

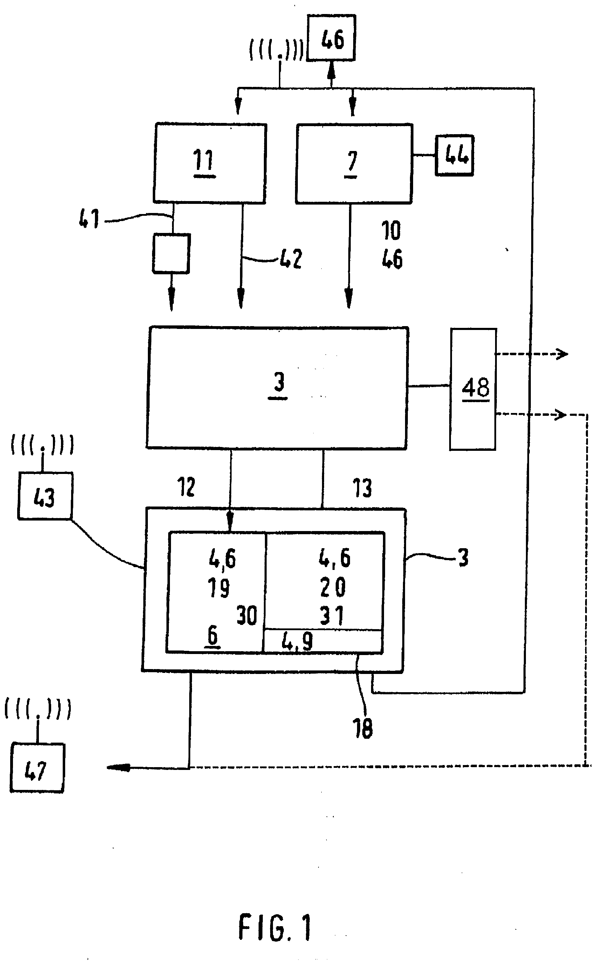

Fig. 1 zeigt die Schritte des erfindungsgemäßen Planungsverfahrens auf, wobei die jeweiligen Schritte nicht notwendig zeitlich nacheinander und in direkter Abfolge vorgenommen werden müssen.

In einem ersten Schritt wählt der Planer/Operateur mehrere verschiedene mehrdimensionale Patientenbilder, insbesondere 2D- und/oder 3D-Patientenbilder **41**, **42**, aus einer externen Bildquelle, dem Speichermedium **11** (beispielsweise eine Festplatte eines externen Rechners, einen USB-Stick, eine CD oder eine DVD oder ein Bildarchiv-Datenprogramm, ein PACS-System eines Krankenhauses, ein medizinisches Bildarchiv oder ein ähnliche feststehendes und/oder mobiles Speichermedium), aus, die der Bildassistenzeinrichtung **3** zugänglich gemacht, beispielsweise in diese überspielt werden.

Dabei sind mehrere, verschiedene mehrdimensionale Patientenbilder, die der präoperativen Planung zugänglich gemacht werden sollen, gleichzeitig auswählbar.

Für die Planung können insbesondere mehrere Patientenbilder, die beispielsweise zu verschiedenen Zeitpunkten entstanden sind und/oder verschiedene Dimensionen darstellen, in die Bildassistenzeinrichtung **3** überspielt werden.

Der Bildassistenzeinrichtung **3** können über das externe Speichermedium **11** zusätzlich weitere Referenzdaten **10**, wie Körpergröße, Gewicht, Alter, Vorerkrankungen des Patienten oder Daten zur bisherigen Krankengeschichte zugänglich gemacht werden.

In einem ersten Zwischenschritt werden die ausgewählten 2D-Patientenbilder, vor, während oder nachdem sie in der Bildassistenzeinrichtung **3** zugänglich gemacht wurden, jeweils bearbeitet, insbesondere skaliert, so dass Abmessungen des abgebildeten Körperabschnitts des Patienten, insbesondere der Wirbelsäule, und/oder der Maßstab der Aufnahme mit Hilfe der Bildassistenzeinrichtung **3** zugänglich gemacht werden.

Die Skalierung kann bei der Aufnahme eines 2D- oder 3D-Patientenbildes manuell, beispielsweise durch Verwenden eines Referenzobjekts, beispielsweise einer Kugel aus einem nicht-strahlendurchlässigen Material, oder halbautomatisch, oder automatisch, also rechnergestützt, erfolgen.
Der Skalierungsvorgang ist für jedes einzelne überspielte Patientenbild gesondert durchführbar und wiederholbar.

Vor während oder nach der Skalierung der einzelnen ausgewählten 2D-Patientenbilder werden ausgewählte volumenbasierten Patientenbilder, also 3D-Patientenbilder in einem zweiten Zwischenschritt der Bildassistenzeinrichtung zugänglich gemacht, also beispielsweise in diese überspielt. Dabei werden insbesondere auch Informationen zu der Skalierung der 3D-Patientenbilder und/oder das Format (beispielsweise CT, MRT, etc.) der 3D-Patientenbilder aus dem Speichermedium **11** in Bildassistenzeinrichtung **3** überspielt.

In einem dritten Zwischenschritt kann die Bildassistenzeinrichtung **3** zur Übernahme von Patienteninformationen, wie z.B. Patientenbilder, Bemaßungsdaten, Funktionsdaten, und/oder sonstiger Referenzdaten **10**, und/oder Informationen zu Implantaten **2**, beispielsweise 2D- und 3D-Modellen **46** von Implantaten **2**, Herstellerinformationen, Artikelnummer, Bemaßung, Material, Verwendungsanweisungen etc., zusätzlich mit einem externen Rechner **7** in Verbindung stehen.

Dabei sind in einem externen Rechner **7** oder in einem PACS-System hinterlegte Referenzdaten und/oder weitere vordefinierte Referenzdaten **10**, insbesondere Ideal- und/oder Referenzmodelle und/oder eine Sammlung von Patientenbildern unterschiedlicher Patienten zu operationsrelevanten Körperabschnitten, beispielsweise der Wirbelsäule, in die Bildassistenzeinrichtung **3** überspielbar. Bei dem externen Rechner **7** kann es sich beispielsweise auch um magnetische, optische oder auf Halbleiterstrukturen basierende Speicher handeln, wie eine Festplatte eines externen Rechners, einen USB-Stick, eine CD oder eine DVD oder ein Archiv-Datenprogramm oder ein ähnliches feststehendes und/oder mobiles Speichermedium.

In einem zweiten Schritt können Volumenpatientenbilder beziehungsweise 3D-Patientenbilder mit Hilfe der Bildassistenzeinrichtung **3** jeweils segmentiert werden, d.h. das jeweilige 3D-Patientenbild wird in einer beispielsweise rechnergestützten, automatischen, halb-automatischen oder manuellen Analyse nach beispielsweise Knochen, insbesondere Wirbelkörpern, abgesucht.

Das abgesuchte Körperteil wird erkannt und mit der medizinisch/anatomisch korrekten Bezeichnung und/oder Bezifferung versehen.

Bei dieser Segmentierung werden beispielsweise Wirbelkörper und Bandscheiben durch eine rechnergestützte Analyse erkannt und hervorgehoben. Dabei werden verschiedene Punkte von Knochen durch Vergleich mit Referenzdaten **10** abgeglichen und optisch markiert mit Hilfe der Bildassistenzeinrichtung, insbesondere auf einem Bildschirm, hervorgehoben. Der Vergleich kann auch rechnergestützt durchgeführt werden.

Die Segmentierung ist für jedes einzelne überspielte 3D-Patientenbild gesondert durchführbar und wiederholbar.

Auf diese Weise kann auf den zugänglich gemachten Patientenbildern festgestellt werden, welche Skelettteile, Knochen, Gewebe, Weichteile etc., bei dem jeweiligen Patienten vorhanden sind, wie diese räumlich angeordnet sind, wie die einzelnen Körperteile medizinisch bezeichnet werden und/oder ggf. ob und welche nativen oder pathologischen Veränderungen gegenüber den Referenzdaten **10** vorliegen. Es ist auch möglich die Lage, Bezeichnung etc. von Skelettteilen, Knochen, Gewebe, Weichteilen etc. in dem jeweiligen Patientenbild mit Hilfe der Darstellung einer Skizze in einem weiteren Bildschirmfenster zu visualisieren.

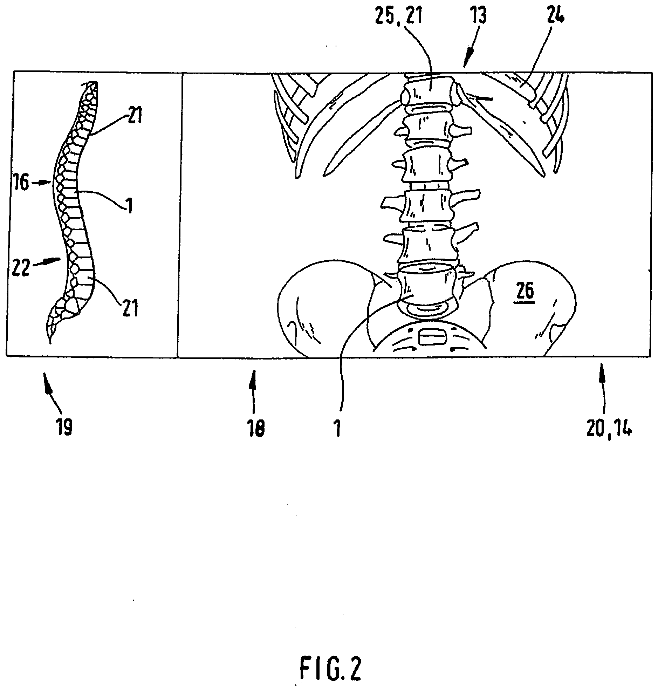

Dies wird beispielhaft in Fig. 2 dargestellt.

Fig. 2 zeigt einen Bildschirm **18** mit zwei Bildschirmfenstern **19** und **20.** Das eine Bildschirmfenster **19** zeigt eine Wirbelsäulenskizze **16** mit Wirbeln **21.** Im unteren Bereich der Wirbel **21** ist ein Bereich **22** der Wirbel **21** dargestellt. Der Bereich **22** der Wirbel **21** ist durch einen nicht dargestellten Cursor markiert.

Im zweiten Bildschirmfenster **20** ist ein segmentierter Wirbel **14** des Körpers **1** (Wirbelsäule) gezeigt, in dem nur die im Bildschirmfenster **19** markierten Wirbel **21** dargestellt sind.

Das Bildschirmfenster **20** zeigt den Körper **1** mit seinen Wirbeln **21** in einer 3D-Darstellung **13**. Im oberen Bereich des Bildschirmfensters **20** ist ansatzweise der Brustkorb **24** dargestellt. Der Brustkorb **24** mit seinen Wirbeln **21** wird über die Wirbelsäule **25** mit dem Becken **26** verbunden.

Eine beispielsweise rechnergestützte, automatische, halbautomatische und/oder manuelle Segmentierung ermöglicht dem Planer/Operateur in einem ersten Zwischenschritt mit Hilfe der Bildassistenzeinrichtung **3** die in einer rechnerbasierten Segmentierung aufgefundenen Ergebnisse, beispielsweise Positionierung von Knochen, insbesondere Wirbeln, Gewebe, Weichteilen etc., deren räumliche Anordnung in den Patientenbilden und/oder deren medizinische Bezeichnung, zu überprüfen.

Es wird dem Planer/Operateur auch ermöglicht, die Segmentierung zu korrigieren, d.h. wenn ein Knochen, insbesondere ein Wirbelkörper, nicht an den im 3D-Patientenbild abgebildeten Konturen rechnergestützt erkannt wurde, die Segmentierung und Bezeichnung manuell in einem zweiten Zwischenschritt etwa mit Hilfe des Cursors **23** nachzubearbeiten.

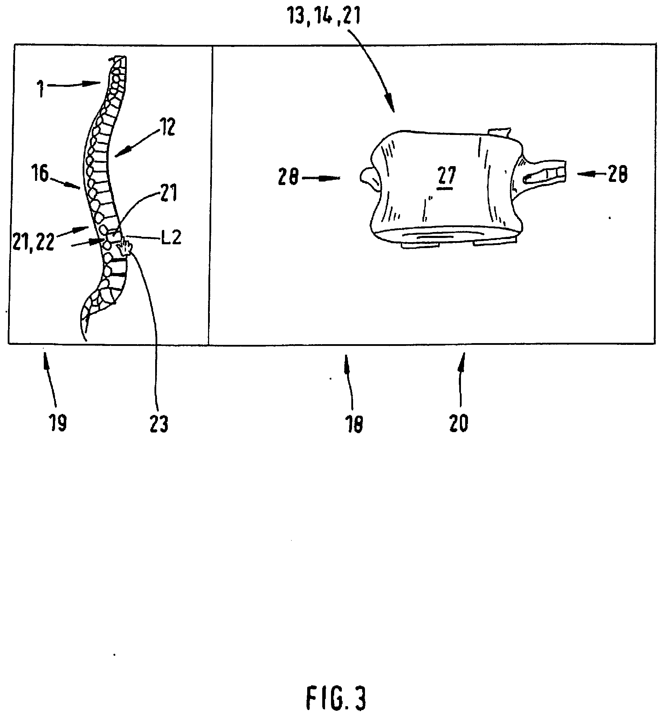

Fig. 3 zeigt hierzu ebenfalls einen Bildschirm **18** mit zwei Bildschirmfenstern **19** und **20**, von denen das Bildschirmfenster **19** eine 2D-Darstellung **12** ist und das benachbarte Bildschirmfenster **20** des Bildschirms **18** einen segmentierten Wirbel **21**, **14** zeigt. Der segmentierte Wirbel ist dabei als 3D-Darstellung **13** gezeigt.

In der Wirbelsäulenskizze **16** ist der Bereich **22** der Wirbel **21** des Körpers **1** markiert. Der Cursor **23** markiert einen bestimmten Wirbel **21**. Der markierte Wirbel **21** ist nun hervorgehoben und farblich anders markiert als die benachbarten Bereiche **22** des Wirbels **21**.

Im Bildschirmfenster **20** des Bildschirms **18** ist der in der Wirbelsäulenskizze **16** markierte Wirbel **21** als 3D-Darstellung **13** dargestellt. Der Wirbel **21** umfasst einen Wirbelkörper **27** und Querfortsätze **28.**

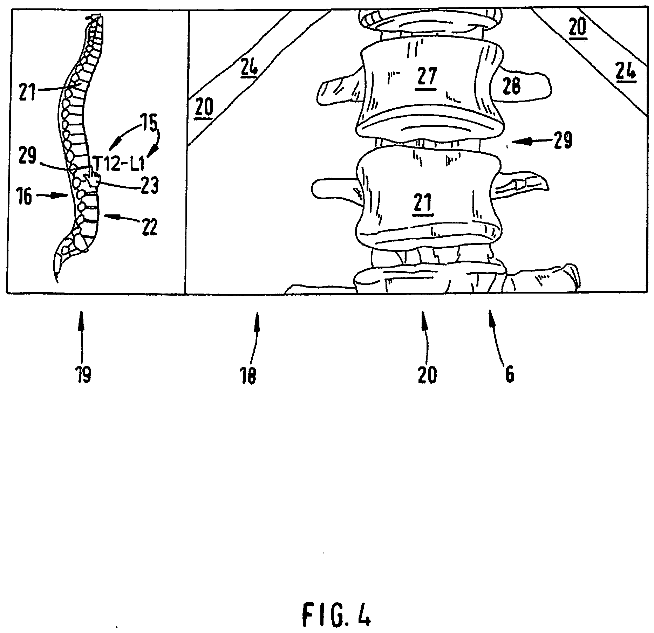

Die Überprüfung der Segmentierung ist durch Markierung von Bereichen der Darstellung von Knochen, Gewebe und Weichteilen, wie in Fig. 4 dargestellt, durchführbar.

Anhand des Vergleichs mit Referenzdaten **10** kann der Planer/Operateur Knochen, insbesondere Wirbel, und Gewebe etc. mit Hilfe des Cursors **23** farblich hervorheben.

Fig. 4 zeigt ein Bildschirmfenster **19** sowie ein Bildschirmfenster **20** des Bildschirms **18** vergleichbar mit der Darstellung aus den Fig. 2 und 3.

Im linken Bildschirmfenster **19** ist die Wirbelsäulenskizze **16** dargestellt, die Wirbel **21** aufweist. Von den Wirbeln **21** ist ein Bereich **22** markiert und vergleichbar mit der Fig. 3 mit einer Bezeichnung **15** "L1" versehen. Der Cursor **23** liegt an der Position der Bandscheibe **29**, die mit der Bezeichnung "T12" versehen ist.

Das zweite Bildschirmfenster **20** ist als Arbeitsdarstellung **6** ausgebildet und zeigt den Wirbel **21** mit Wirbelkörpern **27** und Querfortsätzen **28**.

Entsprechend der Markierung mit Hilfe des Cursors **23** in der Wirbelsäulenskizze **16** ist die Bandscheibe **29** markiert hervorgehoben. Links und rechts der Wirbelkörper **21** sind im Bildschirmfenster **20** Rippen **39** des Brustkorbs **24** dargestellt.

In einem dritten Schritt wird ermöglicht, Knochen, insbesondere Wirbel, Gewebe, Weichteile etc., die in den 2D- bzw. 3D-Patientenbildern dargestellt sind, ortsrichtig durch einen festen Koordinatenbezug zwischen den 2D-Bilddaten und den 3D-Bilddaten, jeweils den 2D- bzw. 3D-Patientenbildern zuzuordnen. Es ergibt sich ein Bezug des jeweiligen Koordinatensystems, so dass zeitgleiche Darstellungen derselben Koordinate in den 2D- bzw. 3D-Patientenbildern in unterschiedlichen Bildschirmfenstern ermöglicht werden.

Der Koordinatenbezug ist rechnergestützt oder manuell herstellbar.

Bei der manuellen Herstellung des Koordinatenbezuges werden beispielsweise mit Hilfe des Cursors **23** Konturlinien von Knochen, insbesondere von Wirbeln wie beispielsweise Grund- und Deckplatten von Wirbelkörpern der Wirbelsäule, auf den 2D- bzw. 3D-Patientenbildern mit Hilfe der Bildassistenzeinrichtung **3** optisch hervorgehoben und beispielsweise farbig markiert. Eine beispielhafte Umreißung von Grund- und Deckplatten von Wirbelkörpern der Wirbelsäule ist in Fig. 4 erkennbar. Dabei werden mit Hilfe der Bildassistenzeinrichtung **3** an jedem Knochenkörper, insbesondere Wirbelkörper, mit Hilfe des Cursors **23** zwei Begrenzungslinien gezeichnet und diese Linien werden zur Herstellung des Koordinatenbezugs mit einer Bezeichnung des betreffenden Wirbelkörpers zugeordnet.

In einem vierten Schritt wählt der Planer/Operateur **43** wenigstens ein 2D- und/oder 3D-Darstellung **12**, **13** als Darstellung in wenigstens einem Bildschirmfenster des Bildschirms **18** der Bildassistenzeinrichtung **3** als Arbeitsdarstellung **6** aus, die zur Fortsetzung der präoperativen Planung verwendet wird.

Die Auswahl wenigstens einer der Darstellungen **4** eines Bildschirmfensters **19**, **20**, **30**, **31** als Arbeitsdarstellung **6** durch den Planer/Operateur **43** ermöglicht, in dieser Darstellung weitere Planungsmaßnahmen, insbesondere Bearbeitungen und/oder Simulationen, durchzuführen.

Die ausgewählten 2D- und/oder 3D-Darstellungen **12**, **13** sind jeweils als Darstellung in verschiedenen Bildschirmfenstern **19**, **20**, **30**, **31** auf einem Bildschirm **18** der Bildschirmassistenzeinrichtung **3** anzeigbar.

Vorzugsweise wählt der Planer/Operateur **43** ein 2D- und ein 3D-Patientenbild aus, zwischen denen ein räumlicher Koordinatenbezug hergestellt wurde. Dies ermöglicht, beispielsweise unterschiedliche Belastungssituationen, insbesondere von Wirbeln und Gelenken, unterschiedliche Haltungen des Patienten, unterschiedliche Aufnahmewinkel und/oder -flächen etc. gleichzeitig darzustellen und so in eine umfassende präoperative Planung einzubeziehen.

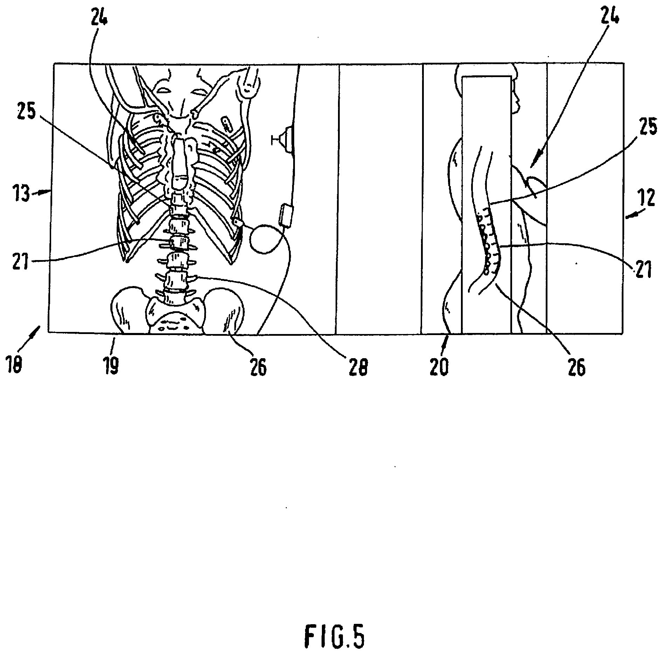

Eine beispielhafte Darstellung findet sich in Fig. 5. Fig. 5 zeigt ein Bildschirmfenster **19** sowie ein Bildschirmfenster **20** des Bildschirms **18** vergleichbar mit der Darstellung aus den Fig. 2 bis Fig. 4. Im linken Bildschirmfenster **19** ist ein 3D-Patientenbild **13** der Wirbelsäule **25** dargestellt, die Wirbel **21** aufweist. Das zweite Bildschirmfenster **20** zeigt ein 2D-Patientenbild der Wirbelsäule **25** den Wirbeln **21**.

Durch den Koordinatenbezug ist beispielsweise eine Messung, Markierung, oder Simulation des Einbringens eines Implantats auf einem 2D- bzw. 3D-Patientenbild, in ein anderes 2D- bzw. 3D-Patientenbild , das mit Hilfe des Cursors **23** ausgeführt wird, rechnergestützt übertragbar, d.h., es wird in wenigstens einer Darstellung in wenigstens einem Bildschirmfenster **19**, **20**, **30**, **31** eine Markierung etc. vorgenommen und diese in einer anderen Darstellung und/oder einem Bildschirmfenster koordinatenbezogen übertragen und dargestellt.

Vorzugsweise kann diese Übertragung bei der Planung von Operationen der Wirbelsäule in sagittaler und axialer Balance, zur Bestimmung von Bandscheibenwinkeln, beispielsweise bei Lordose und Kyphose, vorgenommen werden, wie in Fig. 5 dargestellt.

Eine präoperative Planung ist jedoch auch bei gleichzeitiger Darstellung von unterschiedlichen mehrdimensionalen Patientenbildern ohne Koordinationsbezug in verschiedenen Bildschirmfenstern **19**, **20**, **30**, **31** möglich.

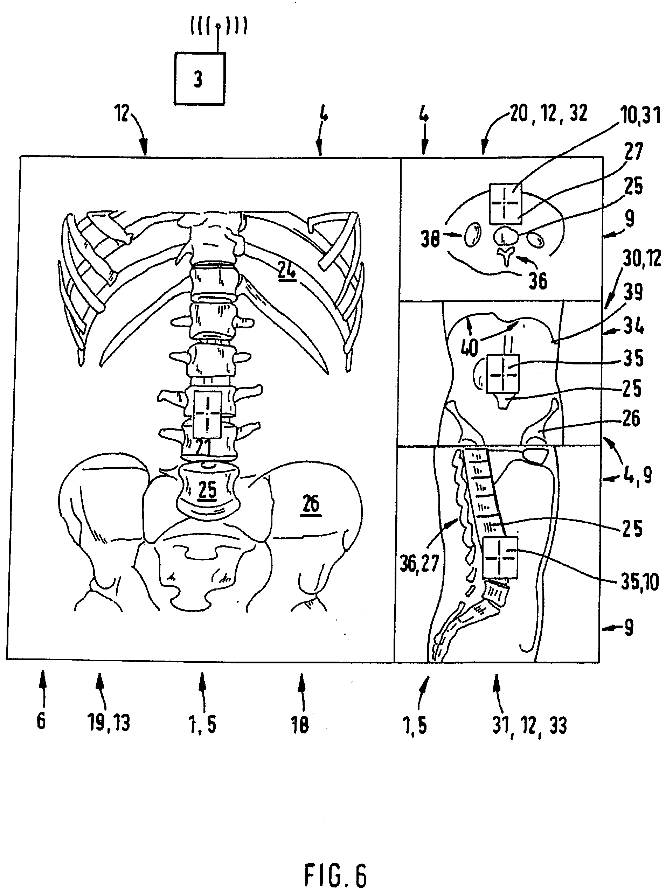

Fig. 6 zeigt hierzu einen Bildschirm **18** mit einem Bildschirmfenster **19** und weiteren Bildschirmfenstern **20**, **30** und **31**.

Das Bildschirmfenster **19** zeigt eine dreidimensionale Darstellung **13** des Körpers **1** mit Brustkorb **24**, Wirbelsäule **25** mit Wirbeln **21** sowie dem Becken **26**. Die Darstellung im Bildschirmfenster **19** des Bildschirms **18** ist die Arbeitsdarstellung **6**.

Die Bildschirmfenster **20**, **30** und **31** sind jeweils dreidimensionale Darstellungen **13**, die je einen axialen Schnitt **32**, einen sagittalen Schnitt **33** und einen koronalen Schnitt **34** durch die Wirbelsäule **25** zeigen.

Mit Hilfe der Bildassistenzeinrichtung **3** kann in den Darstellungen **4** der Bildschirmfenster **19**, **20**, **30**, **31** ein Navigationskreuz **35** angezeigt werden. Das Navigationskreuz **35** umfasst drei Achsen, von denen die eine Achse von oben nach unten verläuft, die zweite Achse von links nach rechts verläuft, sowie eine dritte Achse, die von vorne nach hinten durch den Körper 1 des Patienten verläuft.

Das Bildschirmfenster **20** zeigt die ursprüngliche 3D-Darstellung **13**, aus der die übrigen Darstellungen **4**, **9** der anderen Bildschirmfenster **19**, **30**, **31** errechenbar sind.

Die Darstellung **4**, **9** des Bildschirmfensters **20** zeigt die axiale Darstellung der Wirbelsäule **25** von oben nach unten. An der Rückseite der Wirbelsäule **25** ist ein Dornfortsatz **36** eines Wirbelkörpers **27** gezeigt. Gegenüber dem Dornfortsatz **36** auf der Vorderseite der Wirbelsäule **25** ist die Aorta **37** des Patienten dargestellt. Links und rechts neben der Wirbelsäule **25** sind in dem Bildschirmfenster **20** die Nieren **38** abgebildet.

Die Darstellung **4**, **9** des Bildschirmfensters **30** zeigt den Patienten im Bereich des Navigationskreuzes **35** von vorn gesehen. Links und rechts unterhalb des Navigationskreuzes **35** sind die Knochen des Beckens **26** dargestellt. Im Zentrum der Darstellung **4**, **9** des Bildschirmfensters **30** verläuft von oben nach unten die Wirbelsäule **25**.

Etwa achsparallel zur Wirbelsäule auf einer Linie aus Körpergewebe sind die Ansatzpunkte der Rippen **39** zu erkennen. Zwischen den Rippen **39** sind die Lungenflügel **40** dargestellt.

Im Bildschirmfenster **31** des Bildschirms **18** ist ein sagittaler Schnitt **33** durch die Wirbelsäule **25** gezeigt. Zur Orientierung des Operateurs ist auch im Bildschirmfenster **31** das Navigationskreuz **35** abgebildet. In etwa parallel zu der von oben nach unten verlaufenden Achse des Navigationskreuzes **35** erstreckt sich die Wirbelsäule **25**. Auf der Rückseite der Wirbelsäule **25** sind die Dornfortsätze **36** der Wirbelkörper **27** erkennbar.

In einem fünften Schritt des Verfahrens führt der Planer/Operateur **43** mit Hilfe der Bildassistenzeinrichtung in wenigstens einer Darstellung **6** weitere Planungsmaßnahmen, insbesondere zur Simulation eines zu planenden operativen Eingriffs durch.

Alle Schritte eines zu planenden operativen Eingriffs, insbesondere zum Einsetzen eines Implantats, können in jeder der Darstellungen **4**, **6**, der Bildschirmfenster **19**, **20**, **30**, **31** und/oder der 3D- bzw. 2D-Darstellung **12**, **13** simuliert werden, beispielsweise ein komplettes Ersetzen einer Bandscheibe, das Einsetzen eines Cages und/oder Stents, das Aufzementieren von Knochen, insbesondere von Wirbeln, die Behandlung von Rissen in einer Bandscheibe, Behandlungen von arthritischen Situationen, die Behandlung von Knochenbrüchen, das Einsetzen von Schrauben, insbesondere von Pedikelschrauben, das Einsetzen von Platten, Stegen, etc., das Abschleifen von Knochen, letztlich also der der operativen Methode oder Vorgehensweise. Die Simulation erfolgt durch Auswählen, Abbilden und Bewegen eines 2D- und 3D-Modells **46** eines Implantats **2** mit Hilfe des Cursors **23** in wenigstens einer Arbeitsdarstellung **6**.

Zur Simulation des Eingriffs kann der Planer/Operateur **43** mit Hilfe der Bildassistenzeinrichtung zugänglich gemachte 2D- und 3D-Modelle **46** von Implantaten **2**, beispielsweise Cages (Implantat-Käfige), Bauteile künstlicher Gelenke, Schrauben, Platten, Fixateure, Implantatkomponenten, Ausrichtungsführungen, Einweg-Instrumenten etc., sowie Informationen 46 zu den jeweiligen Implantaten und/oder Befestigungsmitteln für Implantate 2, **46,** beispielsweise Herstellerinformationen, Artikelnummern, Größen, Beschaffenheit, Bebilderung, Bemaßung, Größe, Länge, Durchmesser, Form, Material, Verwendungsanweisungen etc., auswählen.

Die 2D- und 3D-Modelle **46** von Implantaten 2, Informationen zu den jeweiligen Implantaten und/oder Befestigungsmitteln für Implantate können in einer Datenbank, vorzugsweise in dem Rechner **7** oder einem anderen externen Datenspeicher **44**, beispielsweise eine Festplatte eines externen Rechners, einem USB-Stick, einer CD, einer DVD, einem Archiv-Datenprogramm, einem Cloud-System oder einem ähnlichen feststehenden und/oder mobilen Speichermedium gespeichert sein.

Die 2D- und 3D-Modelle **46** von Implantaten **2**, Informationen zu den jeweiligen Implantaten und/oder Befestigungsmitteln für Implantate sind in den Darstellungen **4**, **6**, der Bildschirmfenster **19**, **20**, **30**, **31** und/oder der 3D-bzw. 2D-Darstellung **12**, **13** anzeigbar und bewegbar.

Mit Hilfe der Bildassistenzeinrichtung können insbesondere Bemaßungen, wie etwa Länge, Tiefe, Umfang, Höhe und Breite und/oder Dichte, beispielsweise eines Knochens und/oder Skelettteils, eines Gewebes, eines Weichteils oder Organs, in Form von Referenzpunkten dargestellt werden anhand derer Referenzpunkte an Implantat-Modellen **46**, abgeglichen werden und so automatisch oder manuell passende Implantate, wie Cages (Implantat-Käfige), Bauteile, künstliche Gelenke, Schrauben, Platten, Fixateure, Implantatkomponenten, Ausrichtungsführungen, Einweg-Instrumente, Befestigungsmittel, Pfanne und Schaft für ein Hüftimplantat etc., automatisch ausgewählt vorgeschlagen werden.

In einem vorzugsweise automatischen Auswahlverfahren zur Planung eines operativen Eingriffs wird anhand des Abgleichs von bekannten und/oder ermittelten Referenzpunkten zwischen Implantat-Informationen und den Patientenbildern wenigstens ein am besten passendes Implantat **2** ermittelt, vorgeschlagen und automatisch mit Hilfe der Bildassistenzeinrichtung in eine der Darstellungen, z.B. in das 3D-Patientenbild **13**, eingeblendet.

Die einzelnen Schritte des operativen Eingriffs können so simuliert und/oder dargestellt werden.

Dabei ist unter anderem möglich, ein Implantat, die Position des Implantats im Körper des Patienten, die räumlichen Koordinaten für die Einbringung des Implantats für den Eingriff auszuwählen und den Beginn des Eingriffs sowie die Hilfsmittel zur Befestigung des Implantats und deren räumliche Anordnung festzulegen.

Weiter ist es möglich, sogenannte elektronische Zäune, beispielsweise zur Begrenzung des Operationsbereichs und/oder zur Markierung von bestimmten Bereichen im Körper des Patienten, zur genauen Positionierung eines Implantats in der mehrdimensionalen, vorzugsweise dreidimensionalen Planung, festzulegen und die Informationen über die elektronischen Zäune über eine Schnittstelle an externe Geräte, beispielsweise ein Navigationssystem oder eine Bildschirmassistenzeinrichtung in einem Operationssaal, kabelgebunden oder kabellos zu übermitteln.

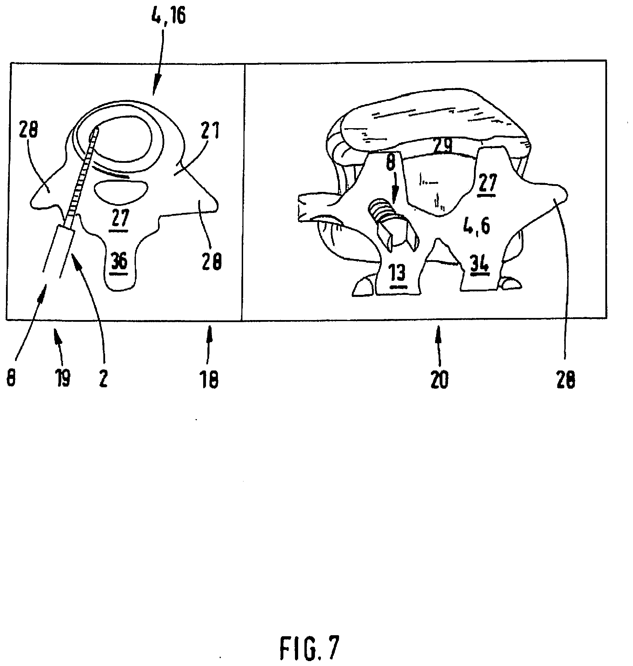

Fig. 7 zeigt hierzu die gleichzeitige Darstellung eines Implantat-Modells, eines Patientenbildes und einer Arbeitsdarstellung bei der Simulation, wie eine Schraube in einen Wirbel eingesetzt werden kann.

Fig. 7 zeigt den Bildschirm **18** mit Bildschirmfenstern **19** und **20.** In diesem Fall ist die Darstellung **4** eine Wirbelkörperskizze. Die Darstellung **4**, **6** ist als dreidimensionale Darstellung **13** im Bildschirmfenster **20** abgebildet.

Die Wirbelsäulenskizze **16** zeigt den Wirbel **21** mit einem Wirbelkörper **27** und den Querfortsätzen **28** sowie einem Dornfortsatz **36.** Im Bereich des linken Querfortsatzes **28** ist ein Implantat-Modell **2**, **46** in Form einer Schraube dargestellt.

Die Positionierung **8** des Implantat-Modells **2** ist in Bezug auf die Ausrichtung, den Anstellwinkel gegenüber dem Wirbelkörper **27** sowie in Bezug auf die Ausbildung der Schraube **2** identisch mit der Positionierung **8** und der Ausbildung der Schraube in der Skizze des Bildschirmfensters **19.**

In den Wirbelkörper **27** ist eine koronale Schnittebene **34** eingezeichnet, an dem die Querfortsätze **28** sowie eine Bandscheibe **29** erkennbar sind.

In einem weiteren optionalen Schritt sind die Planungsschritte eines möglichen zu planenden operativen Eingriffs und/oder das endgültige Planungsergebnis eines operativen Eingriffs jeweils als Datensätze kabellos, beispielsweise über Bluetooth oder sonstige mobile Übermittlungssysteme, oder kabelgebunden speicherbar, insbesondere in dem externen Rechner **7** und/oder in Archivsystemen wie dem Speichermedium **11.**

In einer weiteren optionalen Ausgestaltung des Verfahrens können alle Daten kabellos oder kabelgebunden, auch auf mobile Endgeräte **45** übertragen und dort dargestellt werden.

In einer weiteren vorteilhaften Ausgestaltung des erfindungsgemäßen Verfahrens können die gewonnen Planungsdaten, insbesondere das Planungsergebnis und/oder Teilergebnisse der Planung, über eine Schnittstelle an ein mobiles oder stationäres Operations-Navigationssystem **47** oder eine Bildassistenzeinrichtung übermittelt werden. Die Übermittlung erfolgt kabellos oder kabelgebunden beispielsweise in einen Operationssaal, zur Bestimmung der örtlichen Position und/oder zur Bestimmung der Koordinaten des Eingriffs und der Einleitung von operativen Maßnahmen entsprechend der Planungsdaten.

Dabei ist es insbesondere möglich, die räumliche Anordnung, sogenannte elektronische Zäune, und die zu Grunde liegenden Koordinaten an ein mobiles oder stationäres Operations-Navigationssystem **47** kabellos oder kabelgebunden zu überspielen.

Bei einem solchen Navigationssystem handelt es sich um eine Vorrichtung zur Ausführung eines operativen Eingriffs, der nach dem erfindungsgemäßen Verfahren geplant wurde.

Ein solches Navigationssystem kann insbesondere für die Ausführung eines operativen Eingriffs, der nach dem erfindungsgemäßen Verfahren geplant wurde, verwendet werden.

Auf Grund der Kalibrierung sind die tatsächlichen Abmessungen und Dimensionen des Patienten sind mittels der Bildassistenzeinrichtung gespeichert und können dem Navigationssystem über eine Schnittstelle übermittelt werden.

Instrumente oder Implantate sind beispielsweise aus der verfahrensgemäßen Planung ebenfalls mittels der Bildassistenzvorrichtung gespeichert oder werden mit Hilfe der Bildassistenzeinrichtung zugänglich gemacht, beispielsweise unter Zugriff auf bekannte gespeicherte Informationen oder durch bildgebende und kalibrierte Aufnahmen der Instrumente oder Implantate.

Die Bildassistenzeinrichtung und das Navigationssystem ermöglichen eine automatische Korrelation der Position, Ausdehnung und der Lage des Patienten mit der Position, Ausrichtung und Lage von Implantaten oder Instrumenten.

Zusätzlich können die Instrumente oder Implantate mit wenigstens einem Ortungsmittel verbunden sein, welche die relative Position der Instrumente oder Implantate und Veränderungen in Position, Ausdehnung und Lage der Instrumente oder Apparate an die Bildassistenzeinrichtung und das Navigationssystem übermitteln können. Ortungsmittel können beispielsweise elektromagnetische Ortungssysteme, optische Ortungssysteme, akustische Ortungssysteme sein. Das Ortungsmittel kann zudem Informationen, Signale oder Strahlen kabelgebunden oder kabellos insbesondere an das Navigationssystem oder die Bildassistenzeinrichtung oder an das Navigationssystem und die Bildassistenzeinrichtung senden oder empfangen oder senden und empfangen.

Mit Hilfe der Bildassistenzeinrichtung und des Navigationssystem können alle Werkzeuge, Implantate und die Lage des Patienten relativ zueinander so dargestellt werden, wie sie auch tatsächlich im Raum liegen.

Mit Hilfe der Bildassistenzeinrichtung und des Navigationssystem kann so die Lage, Ausdehnung und Position von verschiedenen Instrumenten und Implantaten, beispielsweise Katheter, Wirbelsäulenimplantate, Proben etc., ermittelt und dargestellt werden. Die Darstellung kann mit Hilfe der Bildassistenzeinrichtung und/oder des Navigationsgerätes sowohl zweidimensional als auch dreidimensional erfolgen. Insbesondere kann die Lage, Ausdehnung und Position dieser Instrumente und Implantate relativ Lage, Ausdehnung und Position des Körpers des Patienten zweidimensional oder dreidimensional dargestellt werden.

Position, Lage und Ausdehnung der Implantate und Instrumente können dabei kontinuierlich oder in zeitlichen Intervallen an die Bildassistenzeinrichtung oder das Navigationssystem oder die Bildassistenzeinrichtung und das Navigationssystem kabelgebunden oder kabellos übermittelt werden.

Insbesondere kann die Bildassistenzeinrichtung verschiedene Koordinaten speichern, darstellen, abrufen und relativ zu anderen Koordinaten darstellen.

Mit Hilfe des patentgemäßen Verfahrens können so Veränderungen in der Position, Lage und Ausrichtung der Instrumente und relativ zum Körper des Patienten dargestellt und überwacht werden. Mit Hilfe der Bildassistenzeinrichtung und des Navigationssystem kann der Operateur überwachen, ob die durchgeführten Operationsschritte mit dem Planungsergebnis und/oder Teilergebnissen der Planung übereinstimmen und ggf. Korrekturen vornehmen.

Die erfindungsgemäße Bildassistenzeinrichtung **3** besitzt vor allem eine Schnittstelle **48**, über die Onlinedaten während des operativen Eingriffs an Anzeige-, Hilfs-, Mess- und/oder bildgebende Aufnahmeeinrichtungen übermittelt werden können.

Die erfindungsgemäße Bildassistenzeinrichtung **3** kann in einer bevorzugten Ausführungsform über die Schnittstelle **48** auch Daten von den oben bezeichneten Einrichtungen abrufen.

Häufig werden während der Durchführung von Operationen mit bildgebenden Verfahren Aufnahmen gemacht und auf herkömmliche Weise im Operationssaal angezeigt. Die erfindungsgemäße Bildassistenzeinrichtung **3** hat nun über die Schnittstelle **48** die Möglichkeit, mit diesen bildgebenden oder bildanzeigenden Einrichtungen Verbindungen herzustellen und dort vorhandene Daten zu übernehmen.

Die Bildassistenzeinrichtung **3** kann die abgerufenen Daten mit den Planungsdaten vergleichen und entstandene Abweichungen feststellen.

Eine bevorzugte Ausführungsform der Bildassistenzeinrichtung **3** ist in der Lage, über die Schnittstelle **48** empfangene Daten in Echtzeit zu verarbeiten, daraus Informationen abzuleiten und diese ebenfalls in Echtzeit an die angeschlossenen Einrichtungen zurückzusenden. Auf diese Weise können Abweichungen des Operationsverlaufs gegenüber der Operationsplanung festgestellt und signalisiert werden.

Solche Abweichungen können beispielsweise unbeabsichtigte Lageäderungen des zu operierenden Patienten, Achsenfehler bei den zu operierenden Knochen oder Knochenteilen oder das Erreichen beziehungsweise Überschreiten der Grenzen zu verbotenen Bereichen, auch als digitale Zäune bezeichnet, sein.

Ebenso kann die Bildassistenzeinrichtung **3** über die Schnittstelle **48** von peripher angeordneten Einrichtungen nach Abschluss einer Operation oder auch nach Einsetzen eines oder mehrerer Implantate und vor dem Verschluss der Operationsöffnung eine Endvermessung zur Feststellung des Operationserfolges durchführen.

Es ist weiter möglich parallel zum Operateur einen zweiten Planer an der Bildassistenzeinrichtung **3** einzusetzen, der den Operationsvorgang beobachtet, dessen Arbeitsschritte oder Arbeitsergebnisse mit den Planungsdaten vergleicht und gegebenenfalls eingreift.

Über die Schnittstelle **48** der Bildassistenzeinrichtung **3** könne auf vorteilhafte Weise weitergehende Möglichkeiten erschlossen werden.

Es kann über die Schnittstelle **48** ein entferntes Anzeigegerät angeschlossen sein, das während einer Operation neben Bildern aus dem Operationssaal auch die Planungsdaten für die Operation und/oder Ergebnisse aus Messungen während der Durchführung der Operation zeigt. Dies kann beispielsweise für Lehrzwecke genutzt werden.

Der Planer/Operateur **43** kann während der Planung Planungsdaten vor Beginn einer Operation an eine im Operationssaal verwendete Navigationseinrichtung **47** übertragen und in einer weiteren Ausgestaltungsform diese auch programmieren.

Der Planer/Operateur **43** kann im Verlauf oder nach Abschluss einer Planung die Gestalt, die Abmessungen und Sonderbedingungen für ein notwendiges Implantat **2** festlegen. Über die Schnittstelle **48** der Bildassistenzeinrichtung kann er durch die Bildassistenzeinrichtung **3** generierte Steuerdaten an einen 3D-Drucker ausgeben, der unter Verwendung der Steuerdaten ein Modell des benötigten Implantats **2** erzeugt.

Die so hergestellten Modelle der Implantate **2** können als Verkörperung durch den Planer/Operateur **43** benutzt werden, sich das Implantat im Original zu veranschaulichen, Änderungen daran vorzunehmen und die Planung der Operation anschließend fortzusetzen.

Es ist ebenso möglich, die durch einen 3D-Drucker erzeugten Modelle für Anschauungs- und Lehrzwecke zu verwenden.

Sofern geeignete Werkstoffe für 3D-Druck verfügbar sind, können auf diese Weise auch Implantate **2** erzeugt werden.

Eine weitere Möglichkeit der Verwendung der Bildassistenzeinrichtung **3** kann darin bestehen, das während der Planung einer Operation ein geeignetes Implantat **2** ausgewählt wird, das jedoch nachgearbeitet werden muss. In einem solchen Fall können über die Schnittstelle **48** der Bildassistenzeinrichtung **3** Steuerdaten an eine spanabhebende Maschine übertragen werden, die nach diesen Steuerdaten eine Nachbearbeitung der Implantats **2** ausführt.

Eine weitere Nutzungsmöglichkeit für die Bildassistenzeinrichtung **3** besteht darin, dass über die Schnittstelle **48** Eingabegeräte an die Bildassistenzeinrichtung **3** angeschlossen werden. Dadurch wird es möglich, dass während einer Operation aus dem Operationssaal Befehle an die Bildassistenzeinrichtung **3** übermittelt werden, diese die Befehle verarbeitet und beispielsweise an angeschlossene periphere Geräte Informationen übermittelt.

Als Eingabegeräte können alle bekannten Möglichkeiten in Betracht kommen. Insbesondere eine im Operationssaal vorhandene Computereinrichtung mit Eingabemöglichkeiten, ein Tabletcomputer, ein Notebook, ein Smartphon oder Einrichtungen, die eine Gestensteuerung ermöglichen.

Es ist dabei nicht notwendig, dass der Planer/Operateur **43** diese Einrichtungen selbst bedient. Allerdings kann er beispielsweise bei Anwendung der Gestensteuerung die Bildassistenzeinrichtung **3** während der Operation veranlassen, bestimmte 2- oder 3-dimensionale Darstellungen des Operationsbereiches auf einem Anzeigegerät darzustellen und beispielsweise deren Lage zu drehen, deren Größe zu ändern, diese farblich hervorzuheben oder ähnliches. In Verbindung mit den oben beschriebenen Möglichkeiten kann der Planer/Operateur **43** sich jederzeit ein Bild über die tatsächlichen Verhältnisse im zu operierenden Bereich machen.

Die Schnittstelle **48** der Bildassistenzeinrichtung **3** kann abhängig von den Erfordernissen anzuschließender peripherer Geräte drahtgebunden oder drahtlos arbeiten. Eine Kombination beider Möglichkeiten ist ebenso möglich.

Die Bildassistenzeinrichtung **3** kann aufgrund der Planungsergebnisse und/oder der Teilergebnisse der Planung Steuerdaten zur direkten Ansteuerung peripherer Geräte erzeugen und kabellos oder kabelgebunden übermitteln.

Es ist ebenso möglich, die Steuerdaten aufgrund der erfindungsgemäßen Planungsergebnisse und/oder Teilergebnisse der Planung unter Beachtung bestimmter Protokollfestlegungen, die für anzuschließende periphere Geräte gelten, zu erzeugen.

Das erfindungsgemäße Verfahren zur Planung, Begleitung, Überwachung und/oder abschließenden Kontrolle eines operativen Eingriffs und die dazu eingesetzte erfindungsgemäße Vorrichtung besitzen in vorteilhafter Weise Möglichkeiten, zu einem vollständig plan-, überwach- und kontrollierbaren Operationsvorgang zu kommen, bei dem es darüber hinaus möglich ist, aus bekannten Bilddaten, Datenbeständen Dritter und unter Anwendung von Vergleichs- und Auswahlprozessen sowohl hinsichtlich der benötigten technischen Mittel, der Voraussetzungen auf Patientenseite und der Voraussetzungen auf Seiten des Implantats allenotwendigen Maßnahmen festzulegen.

### Bezugsziffern:

- 1: Körper
- 2: Implantat
- 3: Bildassistenzeinrichtung
- 4: Darstellung
- 5: Abschnitt
- 6: Darstellung
- 7: Rechner
- 8: Positionierung
- 9: Darstellung
- 10: Referenzdaten und/oder weitere vordefinierte Werte
- 11: Speichermedium
- 12: 2D-Darstellung
- 13: 3D-Darstellung
- 14: segmentierter Wirbel
- 15: Bezeichnung
- 16: Wirbelsäulenskizze
- 17: benachbarter Körperbereich
- 18: Bildschirm
- 19: Bildschirmfenster
- 20: Bildschirmfenster
- 21: Wirbel
- 22: Bereich
- 23: Cursor
- 24: Brustkorb
- 25: Wirbelsäule
- 26: Becken
- 27: Wirbelkörper
- 28: Querfortsatz
- 29: Bandscheibe
- 30: Bildschirmfenster
- 31: Bildschirmfenster
- 32: axialer Schnitt
- 33: sagittaler Schnitt
- 34: koronaler Schnitt
- 35: Navigationskreuz
- 36: Dornfortsatz
- 37: Aorta
- 38: Niere
- 39: Rippe
- 40: Lungenflügel
- 41: 2D-Patientenbild
- 42: 3D-Patientenbild
- 43: Operateur
- 44: externer Datenspeicher
- 45: mobiles Endgerät
- 46: Modell
- 47: mobiles oder stationäres Navigationssystem
- 48: Schnittstelle

## Patentansprüche

1. Verfahren zur Planung, Vorbereitung, Begleitung, Überwachung und/oder abschließenden Kontrolle eines operativen Eingriffs in den menschlichen oder tierischen Körper (1) zum Einsatz wenigstens eines Implantats (2), bei dem mit Hilfe einer Bildassistenzeinrichtung (3) mindestens zwei jeweils mehrdimensionale Darstellungen (4,12, 13) zumindest eines Abschnitts (5) des Körpers (1) gezeigt werden, von denen sich wenigstens zwei Darstellungen (4) in Bezug auf die Anzahl der Dimensionen der Darstellungen (4) unterscheiden, wobei in einem ersten Schritt der Planer/Operateur mehrere verschiedene mehrdimensionale Patientenbilder, insbesondere 2D- und/oder 3D-Patientenbilder (41, 42), aus einer externen Bildquelle auswählt, die der Bildassistenzeinrichtung (3) zugänglich gemacht werden, in einem zweiten Schritt Volumenpatientenbilder mit Hilfe der Bildassistenzeinrichtung (3) jeweils segmentiert werden, wobei die mehrdimensionalen Darstellungen (12, 13) vor, während oder nach ihrer Darstellung mit Hilfe der Bildassistenzeinrichtung (3) erkannt und bearbeitet werden und in einem dritten Schritt ermöglicht wird, Knochen, die in den 2D- bzw. 3D-Patientenbildern dargestellt sind, ortsrichtig durch einen festen Koordinatenbezug zwischen den 2D-Bilddaten und den 3D-Bilddaten, jeweils den 2D- bzw. 3D-Patientenbildern zuzuordnen, wobei dieselben Koordinaten in den 2D- und/oder den 3D Patientenbildern zeitgleich in unterschiedlichen Bildschirmfenstern dargestellt werden, und in einem vierten Schritt der Planer/Operateur (43) wenigstens eine 2D- und/oder 3D-Darstellung (12, 13) als Darstellung in wenigstens einem Bildschirmfenster des Bildschirms (18) der Bildassistenzeinrichtung (3) als Arbeitsdarstellung (6) auswählt, die zur Fortsetzung der präoperativen Planung verwendet wird, in einem fünften Schritt der Planer/Operateur (43) mit Hilfe der Bildassistenzeinrichtung in wenigstens einer Darstellung (6) weitere Planungsmaßnahmen, insbesondere zur Simulation eines zu planenden operativen Eingriffs durchführt, wobei der Bildassistenzeinrichtung Informationen und/oder Daten zu Implantaten (2, 46) und/oder 2D- und/oder 3D-Darstellungen (12, 13) von Implantaten zugänglich gemacht werden und wobei wenigstens ein Patientenbild (12, 13) vor, während oder nach seiner Darstellung mit Hilfe einer Bildassistenzeinrichtung (3) in einer Arbeitsdarstellung erkannt und bearbeitet wird und wobei die wenigstens eine mehrdimensionale Darstellung mit einem Referenzmodell (10) verglichen werden kann und wobei eine Zuordnung der 2D- und 3D-Patientenbilder (12, 13) zueinander erfolgt und wobei alle Schritte eines zu planenden operativen Eingriffs in jeder der Darstellungen (4) und/oder der 3D- bzw. 2D-Darstellung (12, 13) simuliert werden können, und wobei die gewonnenen Planungsdaten über eine Schnittstelle an ein mobiles oder stationäres Operations-Navigationssystem (47) oder eine Bildassistenzeinrichtung übermittelt werden können, wobei die Bildassistenzeinrichtung (3) mit einer Schnittstelle (48) Onlinedaten während des operativen Eingriffs an Anzeige, Hilfs-, Mess- und bildgebende Aufnahmeeinrichtungen übermittelt, und wobei der Bildassistenzeinrichtung (3) zur Planung, Begleitung, Überwachung und/oder abschließenden Kontrolle eines operativen Eingriffs Referenzdaten, Soll-Werte, Standard-Werte und/oder allgemeine patientenunspezifische Daten und/oder Informationen zu Implantaten bereitgestellt werden, welche vor der Bereitstellung einem Abgleich mit Normwerten zugeführt werden.

2. Verfahren nach Anspruch 1, **dadurch gekennzeichnet, dass** mit Hilfe der Bildassistenzeinrichtung vor Beginn der Operation mindestens eine Aufnahme des Patienten in der Anordnung angefertigt wird, die der Patient während der Operation einnimmt, und wobei einer Bildassistenzeinrichtung im Operationssaal über eine Schnittstelle Informationen zur Planung der Operation übergeben werden, und wobei die einzelnen Operationsschritte mit Hilfe der Bildassistenzeinrichtung mit den präoperativ geplanten Operationsschritten abgeglichen werden.

3. Verfahren nach der Patentansprüche 1 oder 2, bei dem mit Hilfe der Bildassistenzeinrichtung (3) an einer oder mehreren Darstellungen (4, 9) des Körperabschnitts (5) wenigstens ein im Verhältnis zu diesem bearbeitbares Implantat-Modell (2, 46) positionierbar ist.

4. Verfahren nach einem der Patentansprüche 1 bis 3, bei dem mit Hilfe der Bildassistenzeinrichtung (3) die Positionierung (8) und/oder Bearbeitung des wenigstens einen Implantat-Modells (2, 46) in Bezug zum Körperabschnitt (5) auf alle oder auf wenigstens eine Darstellungen (4, 9) übertragen und an diesem(n) (4, 9) angezeigt wird.

5. Verfahren nach einem der Patentansprüche 1 bis 4, bei dem die Planungsschritte und/oder Planungsergebnis in der Bildassistenzeinrichtung (3) und/oder in einem sonstigen Speicher (7, 11, 44) gespeichert werden.

6. Verfahren nach einem der Patentansprüche 1 bis 5, bei dem anhand der Ergebnisse der Planung durch die Bildassistenzeinrichtung (3) über die Schnittstelle (48) Steuerdaten an einen 3D Drucker ausgegeben werden.

7. Verfahren nach einem der Patentansprüche 1 bis 6, bei dem die Ergebnisse der Planung und oder Ergebnisse einer interoperativen Erfassung und/oder Ergebnisse einer abschließenden Kontrolle über die Schnittstelle (48) an ein von der Bildassistenzeinrichtung (3) entferntes Anzeigegerät übertragen werden und von einem Bediener interaktiv die Steuerung des angezeigte Bildes übernommen werden kann.

8. Verfahren nach einem der Patentansprüche 1 bis 7, bei dem über Handeingaben bestimmbar ist, welche Planungsinformationen über die Schnittstelle (48) an das Navigationssystem (47) übergeben werden.

9. Verfahren nach einem der Patentansprüche 1 bis 8, bei dem am Implantat Referenzpunkte bestimmbar sind, die Referenzpunkte über die Schnittstelle (48) an das Navigationssystem (47) übergeben und vom Navigationssystem (47) mit den Vorgabewerten verglichen werden.

10. Verfahren nach einem der Patentansprüche 1 bis 9, bei dem das Navigationssystem (47) optische und akustische Signale ausgibt.

11. Verfahren nach einem der Patentansprüche 1 bis 10, bei dem während der Planung einer Operation verbotene Bereiche errechnet, in die Planung eingefügt und während der Operation überprüft und/oder nach der Operation überprüft werden.

12. Verfahren nach einem der Patentansprüche 1 bis 11, bei dem Daten zur Begrenzung des Operationsbereichs und/oder zur Markierung von bestimmten Bereichen im Körper(1) des Patienten und/oder zur genauen Positionierung eines Implantats eingebbar und/oder abrufbar sind.

13. Verfahren nach einem der Patentansprüche 1 bis 12, bei dem eine Mess- und/oder bildgebende Aufnahmeeinrichtung, die über die Schnittstelle (48) Daten online an die Bildassistenzeinrichtung (3) liefert, zugeordnet ist.

14. Vorrichtung zur Durchführung eines Verfahrens nach Patentanspruch 1, die aus einer Bildassistenzeinrichtung (3) mit an dieselbe angepasster Software, einer Schnittstelle (48) zur Übernahme von Onlinedaten an Anzeige-, Hilfs-, Mess- und/oder bildgebenden Aufnahmeeinrichtungen besteht, der über die Schnittstelle (48) eine räumlich entfernte Anzeigeeinrichtung zugeordnet ist, der eine Hilfseinrichtung, die Steuerdaten von der Bildassistenzeinrichtung (3) erhält, zugeordnet ist und die über die Schnittstelle (48) Daten online insbesondere an Navigationsgerät (47) liefert, wobei dieselben Koordinaten in den 2D- und/oder den 3D Patientenbildern zeitgleich in unterschiedlichen Bildschirmfenstern darstellbar sind.

## Claims

1. Process for the planning, preparation, supervision, monitoring and/or final control of a surgical intervention in the human or animal body (1) in order to insert at least one implant (2), in which process at least two in each case multidimensional representations (4, 12, 13) of at least one section (5) of the body (1) are displayed with the aid of an image assistance device (3), of which at least two representations (4) differ with respect to the number of dimensions of the representations (4), wherein in a first step, the planner/surgeon selects a plurality of different multidimensional patient images, in particular 2-D and/or 3-D patient images (41, 42), from an external image source, which patient images are made available to the image assistance device (3), in a second step, volume patient images are each segmented with the aid of the image assistance device (3), wherein the multidimensional representations (12, 13) are recognized and processed before, during or after their representation with the aid of the image assistance device (3), and in a third step, it is made possible to assign bones which are displayed in the 2-D and 3-D patient images, respectively, to the 2-D and 3-D patient images, respectively, in a positionally correct manner by means of a fixed coordinate reference between the 2-D image data and the 3-D image data, wherein the same coordinates in the 2-D and/or 3-D patient images are simultaneously displayed in different screen windows, and in a fourth step, the planner/surgeon (43) selects at least one 2-D and/or 3-D representation (12, 13) as a representation in at least one screen window of the screen (18) of the image assistance device (3) as a working representation (6) which is used to continue the preoperative planning, in a fifth step, the planner/surgeon (43) carries out further planning measures, in particular for simulating a surgical intervention to be planned, with the aid of the image assistance device in at least one representation (6), wherein information and/or data on implants (2, 46) and/or 2-D and/or 3-D representations (12, 13) of implants are made available to the image assistance device, and wherein at least one patient image (12, 13) is recognized and processed before, during or after its representation with the aid of an image assistance device (3) in a working representation, and wherein the at least one multidimensional representation can be compared with a reference model (10), and wherein the 2-D and 3-D patient images (12, 13) are assigned to one another, and wherein all steps of a surgical intervention to be planned can be simulated in each of the representations (4) and/or the 3-D or 2-D representation (12, 13), and wherein the obtained planning data can be transferred via an interface to a mobile or stationary surgical navigation system (47) or an image assistance device, wherein the image assistance device (3) with an interface (48) transfers online data during the surgical intervention to display, auxiliary, measuring and imaging recording devices, and wherein reference data, target values, standard values and/or general patient-unspecific data and/or information on implants are made available to the image assistance device (3) for the planning, supervision, monitoring and/or final control of a surgical intervention, which are matched with standard values before they are made available.

2. Process according to claim 1, **characterized in that** at least one image of the patient in the position which the patient adopts during the operation is made with the aid of the image assistance device before the beginning of the operation, and wherein information for the planning of the operation is transferred to an image assistance device in the operating room via an interface, and wherein the individual operation steps are matched with the preoperatively planned operation steps with the aid of the image assistance device.

3. Process according to claims 1 or 2, wherein at least one implant model (2, 46) which can be processed in relation to the body section (5) can be positioned in one or a plurality of representations (4, 9) of the body section (5) with the aid of the image assistance device (3).

4. Process according to any of claims 1 to 3, wherein, with the aid of the image assistance device (3), the positioning (8) and/or processing of the at least one implant model (2, 46) in relation to the body section (5) is transferred to all or at least one representations (4, 9) and displayed in the latter (4, 9).

5. Process according to any of claims 1 to 4, wherein the planning steps and/or the planning result are stored in the image assistance device (3) and/or in another memory (7, 11, 44).

6. Process according to any of claims 1 to 5, wherein control data are output to a 3-D printer via the interface (48) by the image assistance device (3) on the basis of the results of the planning.

7. Process according to any of claims 1 to 6, wherein the results of the planning and/or the results of an interoperative acquisition and/or the results of a final control are transferred via the interface (48) to a display device which is remote from the image assistance device (3), and the control of the displayed image can be interactively taken over by a user.

8. Process according to any of claims 1 to 7, wherein it is possible to determine via manual inputs which planning information is transferred via the interface (48) to the navigation system (47).

9. Process according to any of claims 1 to 8, wherein reference points can be determined on the implant, the reference points are transferred via the interface (48) to the navigation system (47) and are compared with the default values by the navigation system (47).

10. Process according to any of claims 1 to 9, wherein the navigation system (47) outputs visual and audible signals.

11. Process according to any of claims 1 to 10, wherein during the planning of an operation, prohibited areas are calculated, incorporated into the planning and monitored during the operation and/or monitored after the operation.

12. Process according to any of claims 1 to 11, wherein data for limiting the operating area and/or for marking specific areas in the body (1) of the patient and/or for precisely positioning an implant can be entered and/or retrieved.

13. Process according to any of claims 1 to 12, wherein a measuring and/or imaging recording device which supplies data online via the interface (48) to the image assistance device (3) is assigned.

14. Device for carrying out a process according to claim 1 which comprises an image assistance device (3) with software adapted thereto, an interface (48) for transferring online data to display, auxiliary, measuring and/or imaging recording devices, which is assigned a spatially remote display device via the interface (48), which is assigned an auxiliary device receiving control data from the image assistance device (3), and which supplies data online via the interface (48), in particular to the navigation system (47), wherein the same coordinates can be displayed simultaneously in the 2-D and/or 3-D patient images in different screen windows.

## Revendications

1. Procédé pour la planification, la préparation, l'accompagnement, la surveillance et/ou le contrôle final d'une intervention chirurgicale dans le corps humain ou animal (1) destinée à l'insertion d'au moins un implant (2), dans lequel sont présentées à l'aide d'un dispositif d'assistance par imagerie (3) au moins deux représentations (4, 12, 13), chacune pluridimensionnelle, d'au moins une partie (5) du corps (1), dont au moins deux représentations (4) diffèrent par le nombre des dimensions des représentations (4), dans lequel dans une première étape le planificateur/l'opérateur sélectionne à partir d'une source d'images externe plusieurs images pluridimensionnelles de patient différentes, en particulier des images de patient en 2D et/ou 3D (41, 42), qui sont rendues accessibles au dispositif d'assistance par imagerie (3), dans une deuxième étape des images de patient en volume sont segmentées chacune à l'aide du dispositif d'assistance par imagerie (3), les représentations pluridimensionnelles (12, 13) étant reconnues et traitées à l'aide du dispositif d'assistance par imagerie (3) avant, pendant ou après leur représentation et dans une troisième étape il est rendu possible d'affecter chacun en position exacte aux images de patient en 2D ou 3D les os qui sont représentés dans les images de patient en 2D ou les images de patient en 3D par une relation fixe de coordonnées entre les données d'images en 2D et les données d'images en 3D, ces coordonnées dans les images de patient en 2D et/ou les images de patient en 3D étant représentées simultanément dans des fenêtres d'écran différentes, et dans une quatrième étape le planificateur/opérateur (43) sélectionne au moins une représentation 2D et/ou en 3D (12, 13) comme représentation dans au moins une fenêtre de l'écran (18) du dispositif d'assistance par imagerie (3) en tant que représentation de travail (6) qui est utilisée pour la poursuite de la planification préopératoire, dans une cinquième étape le planificateur/opérateur (43) effectue à l'aide du dispositif d'assistance par imagerie dans au moins une représentation (6) d'autres actions de programmation, en particulier destinées à la simulation d'une intervention chirurgicale à planifier, des informations et/ou des données relatives aux implants (2, 46) et/ou aux représentations en 2D et/ou 3D (12, 13) d'implants étant rendues accessibles au dispositif d'assistance par imagerie et au moins une image de patient (12, 13) étant reconnue et traitée dans une représentation de travail avant, pendant ou après sa représentation à l'aide d'un dispositif d'assistance par imagerie (3) et ladite au moins une représentation pluridimensionnelle pouvant être comparée à un modèle de référence (10) et une affectation des images de patient en 2D et 3D (12, 13) s'effectuant les unes par rapport aux autres et toutes les étapes d'une intervention chirurgicale à planifier pouvant être simulées dans chacune des représentations (4) et/ou de la représentation en 3D ou en 2D (12, 13), et les données de planification acquises pouvant être transmises via une interface à un système de navigation d'opération (47) fixe ou mobile ou à un dispositif d'assistance par imagerie, le dispositif d'assistance par imagerie (3) transmettant à l'aide d'une interface (48) des données en ligne, pendant l'intervention chirurgicale, à des dispositifs d'affichage, auxiliaires, de mesure et d'enregistrement d'imagerie, et au dispositif d'assistance par imagerie (3), pour la planification, l'accompagnement, la surveillance et/ou le contrôle final d'une intervention chirurgicale, des données de référence, des valeurs nominales, des valeurs de référence et/ou des données générales non spécifiques de patient et/ou des informations relatives aux implants étant mises à disposition, qui sont envoyées avant la mise à disposition vers une comparaison avec des valeurs normales.

2. Procédé selon la revendication 1, **caractérisé en ce qu'**avant le début de l'opération au moins un enregistrement du patient est effectué dans la disposition que le patient adopte pendant l'opération, à l'aide du dispositif d'assistance par imagerie, et dans lequel des informations relatives à la planification de l'opération sont transmises via une interface à un dispositif d'assistance par imagerie dans la salle d'opération, et dans lequel les étapes individuelles de l'opération sont comparées, à l'aide du dispositif d'assistance par imagerie, aux étapes de l'opération prévues avant l'opération.

3. Procédé selon l'une quelconque des revendications 1 et 2, dans lequel au moins un modèle d'implant (2, 46) pouvant être traité en rapport avec celui-ci peut être positionné à l'aide du dispositif d'assistance par imagerie (3), sur une ou plusieurs représentations (4, 9) de la partie du corps (5).

4. Procédé selon l'une quelconque des revendications 1 à 3, dans lequel le positionnement (8) et/ou le traitement dudit au moins un modèle d'implant (2, 46) est/sont transcrit(s) à l'aide du dispositif d'assistance par imagerie (3) en relation avec la partie du corps (5) sur l'ensemble ou sur au moins l'une des représentations (4, 9) et affiché(s) sur celle(s)-ci.

5. Procédé selon l'une quelconque des revendications 1 à 4, dans lequel les étapes de planification et/ou les résultats de la planification sont enregistrés dans le dispositif d'assistance par imagerie (3) et/ou dans une autre mémoire (7, 11, 44).

6. Procédé selon l'une quelconque des revendications 1 à 5, dans lequel à l'aide des résultats de la planification, des données de commande sont fournies par le dispositif d'assistance par imagerie (3) via l'interface (48) à une imprimante 3D.

7. Procédé selon l'une quelconque des revendications 1 à 6, dans lequel les résultats de la planification et/ou les résultats d'une acquisition interopérative et/ou les résultats d'un contrôle final sont transmis via l'interface (48) à un appareil d'affichage distant du dispositif d'assistance par imagerie (3) et le réglage de l'image affichée peut être effectué de manière interactive par un serveur.

8. Procédé selon l'une quelconque des revendications 1 à 7, dans lequel il est possible de déterminer par des saisies manuelles quelles informations de planification sont transmises par l'interface (48) au système de navigation (47).

9. Procédé selon l'une quelconque des revendications 1 à 8, dans lequel il est possible de déterminer sur l'implant des points de référence, les points de référence sont transmis par l'interface (48) au système de navigation (47) et sont comparés par le système de navigation (47) aux valeurs prescrites.

10. Procédé selon l'une quelconque des revendications 1 à 9, dans lequel le système de navigation (47) émet des signaux optiques et acoustiques.

11. Procédé selon l'une quelconque des revendications 1 à 10, dans lequel pendant la planification d'une opération des zones interdites sont déterminées par le calcul, intégrées dans la planification, et contrôlées pendant l'opération et/ou contrôlées après l'opération.

12. Procédé selon l'une quelconque des revendications 1 à 11, dans lequel des données relatives à la délimitation du champ opératoire et/ou au marquage de zones déterminées dans le corps (1) du patient et/ou au positionnement précis d'un implant peuvent être saisies et/ou consultées.

13. Procédé selon l'une quelconque des revendications 1 à 12, dans lequel est utilisé un dispositif d'enregistrement d'imagerie et/ou de mesure qui fournit via l'interface (48) des données en ligne au dispositif d'assistance par imagerie (3).

14. Dispositif destiné à la mise en oeuvre d'un procédé selon la revendication 1, qui est constitué d'un dispositif d'assistance par imagerie (3) avec un logiciel adapté à celui-ci, une interface (48) destinée au transfert de données en ligne à des dispositifs d'affichage, auxiliaires, de mesure et/ou d'enregistrement d'imagerie, auquel est affecté via l'interface (48) un dispositif d'affichage spatialement distant, auquel est affecté un dispositif auxiliaire qui acquiert du dispositif d'assistance par imagerie (3) les données de commande et qui fournit via l'interface (48) des données en ligne en particulier à l'appareil de navigation (47), les mêmes coordonnées pouvant être représentées dans les images de patient en 2D et/ou les images de patient en 3D simultanément dans diverses fenêtres d'écran.
